(19) Europäisches Patentamt
European Patent Office
Office européen des brevets

(11) **EP 4 389 215 A1**

(12) **EUROPEAN PATENT APPLICATION**

(43) Date of publication:
**26.06.2024   Bulletin 2024/26**

(21) Application number: **22383245.2**

(22) Date of filing: **20.12.2022**

(51) International Patent Classification (IPC):
***A61P 25/00*** (2006.01)     ***A61P 25/02*** (2006.01)
***A61P 25/28*** (2006.01)     ***G01N 33/68*** (2006.01)

(52) Cooperative Patent Classification (CPC):
**A61P 25/00; A61P 25/02; A61P 25/28;**
**G01N 33/6845; G01N 33/6896;** G01N 2500/20;
G01N 2800/2814; G01N 2800/2835

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Consejo Superior De Investigaciones**
**Científicas**
**28006 Madrid (ES)**

(72) Inventors:
• **CARRASCO CASTRO, Jaime**
  **28006 Madrid (ES)**
• **ANTÓN, Rosa**
  **28006 Madrid (ES)**

• **PANTOJA UCEDA, David**
  **28006 Madrid (ES)**
• **LAURENTS, Douglas V.**
  **28006 Madrid (ES)**
• **GASSET VEGA, María**
  **28006 Madrid (ES)**
• **OROZ GARDE, Francisco Javier**
  **28006 Madrid (ES)**

(74) Representative: **Pons**
**Glorieta Rubén Darío 4**
**28010 Madrid (ES)**

Remarks:
The complete document including Reference
Table(s) and the Sequence Listing(s) can be
downloaded from the EPO website

(54) **CHEMICALLY MODIFIED TDP-43 DERIVED POLYPEPTIDES AND USES THEREOF**

(57)     The present invention refers to polypeptides comprising amino acid sequence SEQ ID NO: 1, wherein, at least, 80% of the methionine are oxidized, and uses thereof in the screening for prophylactic and/or therapeutic compounds for TDP-43 proteinopathies, as detection reagents, as well as *in vitro* diagnosis of TDP-43 proteinopathies.

**EP 4 389 215 A1**

**Description**

The present invention belongs to the field of biomedicine. In particular, it relates to chemically modified TDP-43 polypeptides, and related uses in the screening of prophylactic and/or therapeutic compounds for TDP-43 proteinopathies, such as amyotrophic lateral sclerosis (ALS) or frontotemporal lobar degeneration (FTLD), its application as detection reagents and diagnostic purposes.

**BACKGROUND ART**

Prion-like spreading of aggregated TAR DNA-binding protein 43 (TDP-43) structures plays key roles in the development of amyotrophic lateral sclerosis (ALS), frontotemporal lobar degeneration (FTLD) and other major neurodegenerative diseases (Josephs KA, etal. TDP-43 is a key player in the clinical features associated with Alzheimer's disease. Acta Neuropathol 127, 811-824 (2014); Porta S, et al. Distinct brain-derived TDP-43 strains from FTLD-TDP subtypes induce diverse morphological TDP-43 aggregates and spreading patterns in vitro and in vivo. Neuropathol Appl Neurobiol 47, 1033-1049 (2021); Polymenidou M, Cleveland DW. The seeds of neurodegeneration: prion-like spreading in ALS. Cell 147, 498-508 (2011)). Despite being a vital nuclear DNA and RNA binding protein regulating RNA transcription, splicing, transport and translation, modified TDP-43 forms cytoplasmic pathological inclusions of peculiar morphology under disease (Arseni D, et al. Structure of pathological TDP-43 filaments from ALS with FTLD. Nature 601, 139-143 (2022)).

TDP-43 aggregation is primed by its C-terminal low-complexity domain, that guides the assembly of stress granules by liquid-liquid phase separation (LLPS) in response to cellular stress and also forms prion-like aggregates (Babinchak WM, et al. The role of liquid-liquid phase separation in aggregation of the TDP-43 low-complexity domain. J Biol Chem 294, 6306-6317 (2019)). TDP-43 aggregation arises upon phase de-mixing and transitions from liquid to solid states, which may lead to cell death. Although the nuclear bodies formed by TDP-43 following reversible LLPS are cytoprotective, alternative TDP-43 assemblies formed in the cytoplasm during or in parallel to the aggregation process may be harmful (Alberti S, Hyman AA. Are aberrant phase transitions a driver of cellular aging? Bioessays 38, 959-968 (2016); Fang YS, et al. Full-length TDP-43 forms toxic amyloid oligomers that are present in frontotemporal lobar dementia-TDP patients. Nat Commun 5, 4824 (2014)). Interestingly, whereas the presence of TDP-43 inclusions is often used to define the histopathologic patterns of TDP-43 pathologies, increasing evidence indicates that cytotoxicity is caused by aberrancies in phase de-mixing rather than aggregation (Mateju D, etal. An aberrant phase transition of stress granules triggered by misfolded protein and prevented by chaperone function. EMBO J36, 1669-1687 (2017); Arnold ES, et al. ALS-linked TDP-43 mutations produce aberrant RNA splicing and adult-onset motor neuron disease without aggregation or loss of nuclear TDP-43. Proc Natl Acad Sci USA 110, E736-745 (2013)).

For TDP-43, LLPS is determined by a double $\alpha$-helix signature populated in the C-terminal, prion-like domain (PLD). This particular region, covering the segment 321-343 of TDP-43, evolves into cross $\beta$-sheet structures upon transitions to solid states, forming the core of fibrillar amyloid structures assembled both *in vitro* and *in vivo*. Notwithstanding, TDP-43 present in pathological inclusions is profusely modified (Kametani F, et al. Mass spectrometric analysis of accumulated TDP-43 in amyotrophic lateral sclerosis brains. Sci Rep 6, 23281 (2016)) and knowledge on the impact of modifications on the harmful phase separation and/or amyloid aggregation of TDP-43 is still scarce.

Given the TDP-43 implications in neurodegenerative diseases, different screening and diagnostic approaches based on levels or amounts of said protein have been developed.

WO2013108926A1 discloses a method of screening for a prophylactic and/or therapeutic drug for amyotrophic lateral sclerosis, based on *in vitro* measurement of TDP-43 amount, wherein test compounds that decrease the protein amount of said TDP-43 compared to control is considered as a candidate prophylactic and/or therapeutic drug for amyotrophic lateral sclerosis.

WO2009044119A1 discloses *in vitro* methods for identifying a subject predisposed to, or suffering from, a neurodegenerative disease comprising the examination of TDP-43 levels in a sample. In addition, this patent application also discloses screening methods of compounds for treating/preventing neurodegenerative diseases based on said TDP-43 levels.

Still, it is notoriously difficult to diagnose TDP-43 proteinopathies such as ALS, which entails a critical delay for starting clinical drug trials (Paganoni, S. et al. Amyotroph. Lat. Scler. Fronto. Degener. 2014, 15 (0): 453-6.). Therefore, it is imperative to find efficient biomarkers for early diagnosis and monitoring of the disease.

The measurement of TDP-43 levels might be considered to be a useful biomarker. However, pathological mechanisms underlying TDP-43 proteinopathies seem to be more complex, and therefore TDP-43 protein levels could give only partial information as biomarker. By the same reason, screening methods based on TDP-43 protein levels may have low selectivity in the selection of candidate compounds for treatment/prevention of said pathologies or in the selection of potentially useful detection reagents.

Therefore, in view of the state of the art, there is a need to develop alternative screening approaches and detection procedures and reagents for TDP-43 proteinopathies.

## DESCRIPTION OF THE INVENTION

The present invention relates to TDP-43 chemically modified polypeptides, and uses thereof in the screening of compounds, *in vitro* diagnosis of TDP-43 proteinopathies, such as amyotrophic lateral sclerosis (ALS) or frontotemporal lobar degeneration (FTLD), as well as its application as detection reagents.

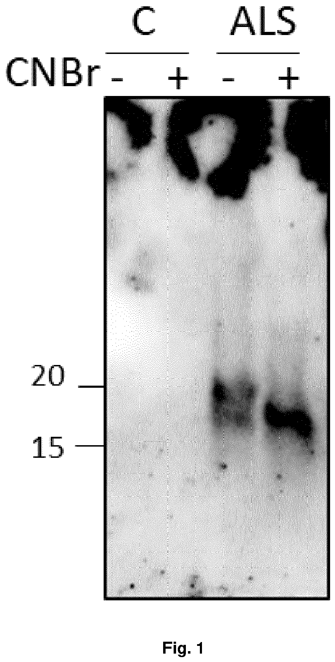

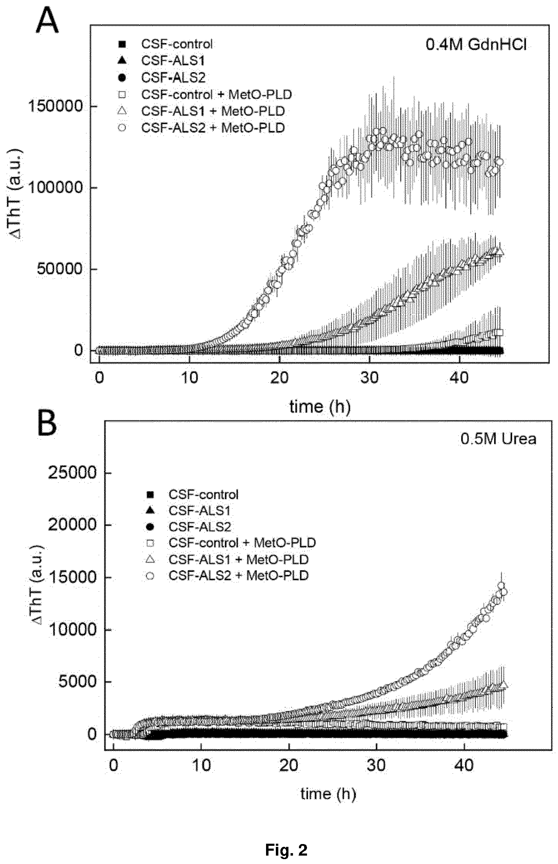

The inventors have generated polypeptides of TDP-43, and fragments thereof, chemically modified by methionine sulfoxidation (MetO). Inventors have shown that methionine sulfoxidized prion-like domain (PLD; the PLD domain referred as PLD-MetO in some parts of the document when having methionine residues oxidized) of TDP-43 commands mechanisms with relevance in proteinopathies, and have demonstrated that it is present in patients with amyotrophic lateral sclerosis (Fig. 1) and the ability of said methionine sulfoxidized TDP-43 derived protein for the interaction with and amplification of pathological aggregates of TDP-43 present in biological fluids (Fig. 2), developing different uses for this MetO TDP-43 peptides.

### Polypeptides of the invention

Thus, an aspect relates to a polypeptide which comprises an amino acid sequence comprising SEQ ID NO: 1, wherein, at least, 80% of the methionine residues are oxidized, hereinafter also named as the "MetO-PLD309 polypeptide of the invention" or simply as "MetO PLD$_{309}$".

SEQ ID NO 1:

GGMNFGAFSINPAMMAAAQAALQSSWGMMGMLASQQNQSGPS

SEQ ID NO: 1 corresponds to the amino acid sequence of a protein fragment of human TDP-43, in particular amino acids 309-350 of human TDP-43.

In a preferred embodiment of the MetO-PLD309 polypeptide of the invention, the amino acid sequence consists of SEQ ID NO: 1, wherein, at least, 80% of the methionine residues are oxidized.

A preferred embodiment, alone or in combination with the rest of preferred embodiments, provides the MetO-PLD309 polypeptide of the invention wherein at least, 85, 90, 91, 92, 93, 94, 95, 96, 97, 98 or 99% of the methionine residues of SEQ ID NO: 1 are oxidized.

In a preferred embodiment, alone or in combination with the rest of preferred embodiments, the oxidized methionine residues of SEQ ID NO: 1 are methionine residues corresponding to M311, M322, M323, M336, M337 and M339 relative to the full human TDP-43 protein amino acid sequence (SEQ ID NO: 3).

Another preferred embodiment provides the MetO-PLD309 polypeptide of the invention wherein all the methionine residues of SEQ ID NO: 1 are oxidized.

In the present invention, the phrase "methionine residues are oxidized", refers to chemically modified methionine residues of an amino acid sequence, by methionine sulfoxidation (MetO) which transforms methionine sulfur atoms into sulfoxide groups. Methionine sulfoxidation (or "methionine oxidation" terms interchangeably used in the present document) can be achieved in the present invention by the incubation of the TDP-43 or related fragments, with $H_2O_2$, as explained in Example 1.5. *PLD covalent modifications.*

Polypeptides can be obtained by techniques or methods known in the state of the art. Examples of techniques for polypeptide obtention include, without limitation to, chemical or biological synthesis, genetic recombination or expression of polynucleotides coding for the polypeptide of interest. In the present invention, the polypeptides of the invention can be obtained by techniques or methods as the ones above mentioned, and subsequent methionine sulfoxidation.

Besides, methods and techniques to assess methionine sulfoxidation of a protein or fragments thereof are known in the state of the art, for instance, but without limitation to, mass spectrometry, Nuclear Magnetic Resonance (NMR) spectroscopy, X-ray crystallography.

Inventors have also generated other methionine oxidized TDP-43 polypeptides, wherein SEQ ID NO:1 is comprised.

Thus, in other preferred embodiment, the polypeptide of the invention comprises the amino acid sequence SEQ ID NO: 2, wherein, at least, 80% of the methionine residues in said amino acid sequence SEQ ID NO: 2 are oxidized, hereinafter also named as "MetO-PLD polypeptide of the invention".

SEQ ID NO: 2

GRFGGNPGGFGNQGGFGNSRGGGAGLGNNQGSNMGGGMNFGAFSINPAMMA

AAQAALQSSWGMMGMLASQQNQSGPSGNNQNQGNMQREPNQAFGSGNNSYS

GSNSGAAIGWGSASNAGSGSGFNGGFGSSMDSKSSGWGM

SEQ ID NO: 2 corresponds to the prion-like domain (PLD) amino acid sequence of the human TDP-43 protein (TDP-43$_{274-414}$).

In a preferred embodiment of the MetO-PLD polypeptide of the invention, the amino acid sequence consists of SEQ ID NO: 2, wherein, at least, 80% of the methionine residues are oxidized.

A preferred embodiment provides the MetO-PLD polypeptide of the invention wherein at least, 85, 90, 91, 92, 93, 94, 95, 96, 97, 98 or 99% of the methionine residues of SEQ ID NO: 2 are oxidized.

In a preferred embodiment, alone or in combination with the rest of preferred embodiments, the oxidized methionine residues of SEQ ID NO: 2 correspond to methionine residues M307, M311, M322, M323, M336, M337, M339, M359, M405 and M414 relative to the full human TDP-43 protein amino acid sequence (SEQ ID NO: 3).

Other preferred embodiment provides the MetO-PLD polypeptide of the invention wherein all the methionine residues of SEQ ID NO: 2 are oxidized

Other polypeptide of the invention, hereinafter also named as "full length MetO-TDP-43 polypeptide of the invention", comprises the amino acid sequence SEQ ID NO: 3, wherein at least, 80% of the methionine residues in the SEQ ID NO: 1 (TDP-43$_{309-350}$), which is comprised by SEQ ID NO: 3, are oxidized, or wherein at least, 80% of the methionine residues in the SEQ ID NO: 2 (TDP-43$_{274-414}$), which is comprised by SEQ ID NO: 3, are oxidized.

Thus, a preferred embodiment provides a polypeptide comprising the amino acid sequence SEQ ID NO: 3, which in turn comprises SEQ ID NO: 1, wherein, at least, 80% of the methionine residues of SEQ ID NO: 1 are oxidized.

Other preferred embodiment provides a polypeptide comprising the amino acid sequence SEQ ID NO: 3, which in turn comprises SEQ ID NO: 2, wherein, at least, 80% of the methionine residues of SEQ ID NO: 2 are oxidized.

SEQ ID NO: 3

MSEYIRVTEDENDEPIEIPSEDDGTVLLSTVTAQFPGACGLRYRNPVSQCMRGVRL

VEGILHAPDAGWGNLVYVVNYPKDNKRKMDETDASSAVKVKRAVQKTSDLIVLGL

PWKTTEQDLKEYFSTFGEVLMVQVKKDLKTGHSKGFGFVRFTEYETQVKVMSQR

HMIDGRWCDCKLPNSKQSQDEPLRSRKVFVGRCTEDMTEDELREFFSQYGDVM

DVFIPKPFRAFAFVTFADDQIAQSLCGEDLIIKGISVHISNAEPKHNSNRQLERSGRF

GGNPGGFGNQGGFGNSRGGGAGLGNNQGSNMGGGMNFGAFSINPAMMAAAQ

AALQSSWGMMGMLASQQNQSGPSGNNQNQGNMQREPNQAFGSGNNSYSGSN

SGAAIGWGSASNAGSGSGFNGGFGSSMDSKSSGWGM

SEQ ID NO: 3 corresponds to the full amino acid sequence of human TDP-43 protein (uniport.org: Q13148 · TADBP_HUMAN).

In a preferred embodiment of the full length MetO-TDP-43 polypeptide of the invention, the amino acid sequence consists of SEQ ID NO: 3, wherein at least, 80% of the methionine residues in the SEQ ID NO: 1 (TDP-43$_{309-350}$), which is comprised by SEQ ID NO: 3, are oxidized, or wherein at least, 80% of the methionine residues in the SEQ ID NO: 2 (TDP-43$_{274-414}$), which is comprised by SEQ ID NO: 3, are oxidized.

A preferred embodiment provides the full length MetO-TDP-43 polypeptide of the invention wherein at least, 85, 85, 90, 91, 92, 93, 94, 95, 96, 97, 98 or 99% of the methionine residues of SEQ ID NO: 1 are oxidized, or wherein at least, 85, 85, 90, 91, 92, 93, 94, 95, 96, 97, 98 or 99% of the methionine residues in the SEQ ID NO: 2 are oxidized.

In a preferred embodiment, alone or in combination with the rest of preferred embodiments, the oxidized methionine residues of SEQ ID NO: 3 are methionine residues M307, M311, M322, M323, M336, M337, M339, M359, M405 and M414.

Other preferred embodiment, alone or in combination with the rest of preferred embodiments, provides the full length MetO-TDP-43 polypeptide of the invention wherein at least, 80, 85, 90, 91, 92, 93, 94, 95, 96, 97, 98 or 99% of the methionine residues of SEQ ID NO: 3 are oxidized.

In the context of the present invention, peptides derived from the peptide of the invention, so-called "variants", are also contemplated, which although they do not have 100% sequence identity with the amino acid sequence SEQ ID NO: 1, SEQ ID NO: 2, or SEQ ID NO: 3, retain its properties as the amino acids have been replaced by biologically similar ones.

In the present invention, "sequence identity" means the degree of similarity between two amino acid sequences obtained by aligning the two sequences. Depending on the number of common residues between the aligned sequences, a degree of identity expressed as a percentage will be obtained. The degree of identity between two amino acid sequences can be determined by conventional methods, e.g. by standard sequence alignment algorithms known in the state of the art, such as BLAST [Altschul S.F. et al. Basic local alignment search tool. J Mol Biol. 1990 Oct 5; 215(3):403-10]. BLAST programs, e.g. BLASTN, BLASTX, and TBLASTX, BLASTP and TBLASTN, are in the public domain on the website of The National Center for Biotechonology Information (NCBI).

Thus, other preferred embodiment, provides a polypeptide comprising an amino acid sequence with at least, 80, 85, 90, 91, 92, 93, 94, 95, 96, 97, 98 or 99% of sequence identity to SEQ ID NO: 1, wherein, at least, 80, 85, 90, 91, 92, 93, 94, 95, 96, 97, 98 or 99% of the methionine residues in said amino acid sequence are oxidized.

Other preferred embodiment provides a polypeptide comprising an amino acid sequence with at least, 80, 85, 90, 91, 92, 93, 94, 95, 96, 97, 98 or 99% of sequence identity to SEQ ID NO: 2, wherein, at least, 80, 85, 90, 91, 92, 93, 94, 95, 96, 97, 98 or 99% of the methionine residues in said amino acid sequence are oxidized.

Other preferred embodiment provides a polypeptide comprising an amino acid sequence with at least, 80, 85, 90, 91, 92, 93, 94, 95, 96, 97, 98 or 99% of sequence identity to SEQ ID NO: 3, which in turn comprises SEQ ID NO: 1, wherein, at least, 80, 85, 90, 91, 92, 93, 94, 95, 96, 97, 98 or 99% of the methionine residues in said amino acid sequence SEQ ID NO: 1, are oxidized.

Other preferred embodiment provides a polypeptide comprising an amino acid sequence with at least, 80, 85, 90, 91, 92, 93, 94, 95, 96, 97, 98 or 99% of sequence identity to SEQ ID NO: 3, which in turn comprises SEQ ID NO: 2, wherein, at least, 80, 85, 90, 91, 92, 93, 94, 95, 96, 97, 98 or 99% of the methionine residues in said amino acid sequence SEQ ID NO: 2, are oxidized.

A preferred embodiments provides any of the polypeptides of the invention, wherein said polypeptide or a fragment of it is arranged in a helicoidal structure.

Another preferred embodiment provides any of the polypeptides of the invention, wherein said polypeptide or a fragment of it is arranged in oligomer and/or polymer structures rich in β-sheets.

In addition, any of the polypeptides of the invention may be labelled or coupled to an indicator. In the present invention, the term "indicator" refers to any compound or molecule capable of being visualized and/or quantified. The term "indicator" may be interchanged with compound or molecule "indicator", "signal", "label" or "reporter".

Examples of indicators include, but are not limited to, dyes, fluorophores, substances with redox activity, plasmonic resonance or biologically active such as cytotoxic agents, proteins, small biomolecules, enzymes or nucleic acid fragments.

Thus, a preferred embodiment provides any of the polypeptides of the invention, which further comprises an indicator. More preferably, the indicator is selected from a list consisting of dyes, substances with redox activity, plasmonically resonant and biologically active substances.

In the present document, the term "polypeptide of the invention", which may be also named as "MetO polypeptides of the invention" or simply as "polypeptides of the invention", is used to refer any of the polypeptides above described, i.e. the "MetO-PLD309 polypeptide of the invention", the "MetO-PLD polypeptide of the invention", the "full length MetO-TDP-43 polypeptide of the invention", as well as any of its variants. As it was described, the polypeptide/s of the invention comprises or consists of an amino acid sequence SEQ ID: 1, SEQ ID NO: 2 (which in turn comprises SEQ ID NO: 1) or SEQ ID NO: 3 (which in turn comprises SEQ ID: NO 2, and therefore, SEQ ID NO: 1), with a given proportion of methionine residues sulfoxidized.

Methods and uses of the invention

*Screening use and method based on MetO polypeptide of the invention* as a *target*

As previously described, the polypeptides of the invention (as explained in the first aspect of the invention, the polypeptides of the invention comprise or consists of TDP-43 amino acid sequences with oxidized methionine residues) have demonstrated its utility in the screening of compounds for treating and/or preventing a TDP-43 proteinopathy. As shown in Examples section, inventors have demonstrated that the TDP-43 prion-like domain (PLD) with oxidized methionine residues commands mechanisms with relevance in said TDP-43 proteinopathies. Moreover, inventors have also demonstrated that methionine sulfoxidized TDP43 is present in samples from ALS patients (Fig. 1).

Therefore, MetO polypeptides of the invention have applications in the screening of compounds, wherein said compounds are useful for treating and/or preventing a TDP-43 proteinopathy. Screening uses of TDP-43 (not chemically modified) based on TDP-43 levels are known in the state of the art, for instance, as described in WO2013108926A1 or WO2009044119A1. However, the screening uses of the present invention are based on the use of methionine sulfoxidized TDP-43 polypeptides instead of TDP-43 levels. Thus, inventors provide alternative targets for screening approaches.

Screening uses of the invention have, as of the main advantages, a higher selectivity in the screening of compounds useful for treating and/or preventing a TDP-43 proteinopathy due to the presence of oxidized methionine residues, which are associated to pathological states. Thus, generated MetO polypeptides of the invention can be used in the screening of compounds, wherein the test compounds which interact with these MetO polypeptides of the invention are candidate compounds for treating and/or preventing a TDP-43 proteinopathy.

Thus, an aspect of the invention relates to the use of any of the MetO polypeptides of the invention as a pharmacological (therapeutic) target for designing and/or screening molecules, compounds, compositions, drugs or the like, useful for the treatment and/or prevention of a TDP-43 proteinopathy, hereinafter the "target MetO polypeptides screening use".

The term "pharmacological or therapeutic target", as used in the present invention, relates to any of the MetO polypeptides of the invention (which as it has been previously explained and defined, comprise oxidized methionine residues), which are useful for studying the biochemical effect of molecules capable of binding and/or neutralizing the activity of the same. The molecules, compounds, compositions, drugs or the like, of interest in the present aspect are those that bind to the MetO-polypeptide of the invention. These molecules may be, for example, antibodies, antigen binding fragments of antibodies, aptamers, a protein, a peptide, a peptidomimetic, a small molecule, a chemical agent or the like. Techniques and methods to evaluate the interaction of compounds/molecules are known in the state of the art, including, without limitation to, immunoblotting, fluorescence spectroscopy, fluorescence microscopy, NMR spectroscopy, thermophoresis, surface plasmon resonance spectroscopy, chemical crosslinking, calorimetry, X-ray crystallography.

As used herein, "TDP-43 proteinopathies" relates to nervous system diseases, in particular, neurodegenerative diseases, which encompass a heterologous group of disorders linked by the TDP-43 protein. These diseases are characterized by the prion-like spreading of aggregated TDP-43 structures. TDP-43 proteinopathies include a wide range of neurodegenerative diseases and phenotypes. Examples of TDP-43 proteinopathies are, without limitation to, ALS, FTLD, ALS-FTLD, Alzheimer's disease, Limbic- predominant age-related TDP-43 encephalopathy or Parkinson's disease.

Thus, in a preferred embodiment, the TDP-43 proteinopathy is selected from the list consisting of amyotrophic lateral sclerosis (ALS), frontotemporal lobar degeneration (FTLD), ALS-FTLD, Alzheimer's disease, Limbic- predominant age-related TDP-43 encephalopathy and Parkinson's disease.

In a more preferred embodiment, the TDP-43 proteinopathy is amyotrophic lateral sclerosis (ALS).

In another more preferred embodiment, the TDP-43 proteinopathy is frontotemporal lobar degeneration (FTLD).

In other aspect, the invention refers to an *in vitro* method of screening for a therapeutic agent useful for treating and/or preventing a TDP-43 proteinopathy, hereinafter, the "target based-screening method of the invention", which comprises:

a) contacting any of the polypeptides of the invention with a therapeutic agent to be tested; and
b) detecting the binding of the therapeutic agent to the MetO polypeptide/s of the invention,

wherein the therapeutic agent is a candidate to be useful for treating and/or preventing a TDP-43 proteinopathy if such binding is detected.

The term "TDP-43 proteinopathy" has been previously explained and its definition as well as its preferred embodiments apply equally to the present aspect of the invention.

Techniques and methods to evaluate the binding or interaction of compounds/molecules are known in the state of the art, such as, without limitation to, immunoblotting, fluorescence spectroscopy, fluorescence microscopy, NMR spectroscopy, thermophoresis, surface plasmon resonance spectroscopy, chemical crosslinking, calorimetry, X-ray crystallography.

Based on the same principle, other application of the MetO polypeptides of the invention relates to the screening of detection reagents of methionine oxidized TDP-43 proteins and/or fragments thereof.

Thus, other aspect of the present invention refers to the use of any of the MetO polypeptides of the invention as a target for designing and/or screening molecules, compounds, compositions, drugs or the like, useful for the detection of methionine oxidized TDP-43 proteins and/or fragments thereof. In a preferred embodiment, the molecule is an antibody.

In other aspect, the invention relates to an *in vitro* method of screening for a detection reagent useful for the detection of methionine oxidized TDP-43 proteins and/or fragments thereof, which comprises:

a) contacting any of the polypeptides of the invention with an agent to be tested; and
b) detecting the binding of the agent to the polypeptide/s of the invention,

wherein the agent is a candidate to be useful for the detection of methionine oxidized TDP-43 proteins and/or fragments thereof if such binding is detected.

In a preferred embodiment, the detection reagent is an antibody.

*Screening method based on pathological TDP-43 aggregates amplification mediated by MetO polypeptides of the invention*

In addition, inventors have demonstrated the ability of polypeptides of the invention to interact with and amplify pathological aggregates of TDP-43 by a seeded conversion. Abnormal aggregation of TDP-43, for instance in neurons and glia, and its accumulation in cerebrospinal fluid is a defining pathological hallmark of TDP-43 proteinopathies. MetO polypeptides of the invention shows specific reactivity in human samples, being able to amplify TDP-43 seeds or propagons, allowing testing the presence of TDP-43 pathological aggregates in a subject sample.

Thus, the polypeptides of the invention, also named as MetO polypeptides of the invention, work as stable, homogeneous and reproducible substrates for amplification. In order to test if a substance meets with these conditions, and therefore testing if it serves as a reliable detection reagent based on amplification of TDP-43 seeds, it is important not only to demonstrate that there is a differential response between subject with a TDP-43 proteinopathy samples and controls (healthy subject samples), but also to demonstrate that the response in the presence of biological material (i.e. material obtained from the subject such as an isolated biological sample, for instance non-haemolytic cerebrospinal fluid) is different from the aggregation of the protein alone.

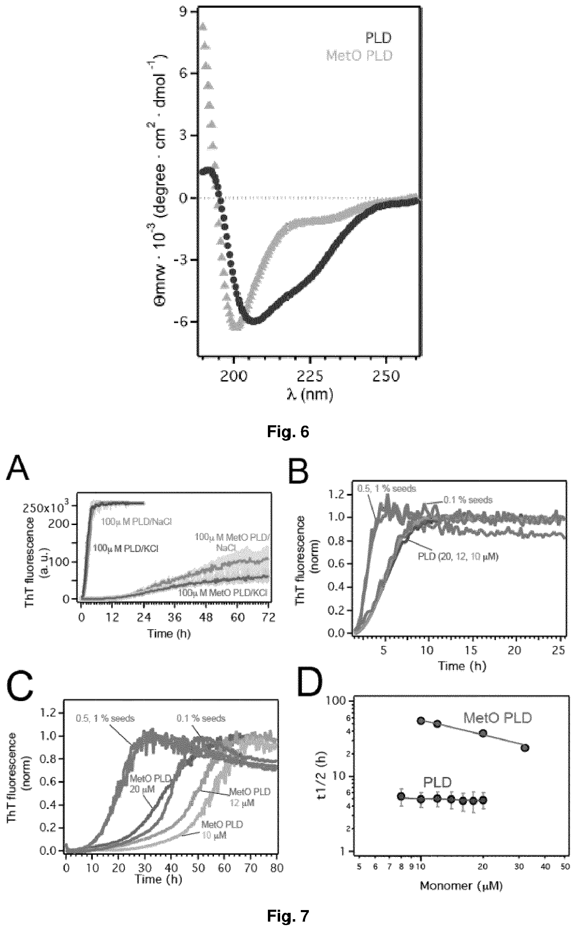

Inventors have demonstrated that when the PLD polypeptide with sulfoxidized methionine residues (MetO-PLD polypeptide of the invention) is used, in presence of pathological biological material, its aggregation is significantly accelerated, especially in the presence of HClGdn, with respect to when the polypeptide of the invention is used in presence of control biological material (Figure 2A and 2B). Furthermore, amplification based on MetO PLD rather than unmodified PLD shows advantages, working as a stable, homogeneous and reproducible substrate for amplification. This is because the aggregation of MetO PLD *per se* follows a distinct mechanism compared to unmodified PLD, with nucleation as the limiting kinetic step in the aggregation process (Fig. 7A), which becomes an advantage in this regard.

Thus, an aspect of the present invention relates to the use of any of the polypeptides of the invention as an amplification substrate of TDP-43 pathological propagons for designing and/or screening molecules, compounds, compositions, drugs or the like, useful for the treatment and/or prevention of a TDP-43 proteinopathy, hereinafter the "amplification-based screening use of the invention".

The term "propagons", synonymous with "seeds", refers to TDP-43 polypeptide aggregates, including oligomer and polymer species, that are able to interact with the monomer polypeptide and convert it into a like.

Other aspect of the present invention refers to a method for the screening of prophylactic and/or therapeutic compounds for a TDP-43 proteinopathy, hereinafter the "amplification-based screening method of the invention", which comprises the following steps:

a) preparing a first reaction mixture, wherein said reaction mixture comprises a previously isolated biological sample from a biological system treated with a test compound, and an amyloid-specific probe, wherein said probe is able to produce a detectable signal;

b) preparing a second reaction mixture, wherein said reaction mixture comprises a previously isolated biological sample from a biological system not treated with a test compound, and an amyloid-specific probe, wherein said probe is able to produce a detectable signal;

c) adding any of the polypeptides of the invention to the reaction mixture prepared in step a), obtaining a reaction mixture (i), and to the reaction mixture prepared in step b), obtaining a control reaction mixture (ii);

d) an amplification step, which comprises incubating with agitation the reaction mixture (i), and the control reaction mixture (ii), preferably, wherein said agitation is carried out in presence of beads;

e) measuring, simultaneously to when the amplification step d) is performed, the detectable signal in the reaction mixture (i) and the control reaction mixture (ii); and

f) selecting, as a prophylactic and/or therapeutic candidate compound for a TDP-43 proteinopathy, a test compound that changes the measured signal in the reaction mixture (i) compared to the measured signal in the control reaction mixture (ii).

As mentioned above, the control reaction mixture ii) is a reaction mixture which comprises, apart from other elements, a previously isolated biological sample from a biological system not treated with a test compound. Therefore, it serves as a control condition (absence of such compound).

In the present method of the invention, the term "test compound" is any chemical, molecule, agent for which it is intended to evaluate whether it is a prophylactic and/or therapeutic candidate compound for a TDP-43 proteinopathy.

The test compound selected as prophylactic and/or therapeutic candidate compound for a TDP-43 proteinopathy may prevent the amplification reaction by interaction with MetO-PLD, the biological sample or both, causing a change in the measured signal compared to a control condition.

Thus, the present aspect of the invention, is directed to the screening of compounds that are candidates for treating and/or preventing a TDP-43 proteinopathy.

The phrase "simultaneously to when the amplification step d) is performed", as used herein, means that the step e) is performed at the same time that step d).

Amyloid-specific probes are known in the state of the art. Such probes are specific and able to produce a detectable signal, such as a fluorescence signal, but without limitation to it. Examples of amyloid-specific probes which can be used in the present invention, include, without limitation to, thioflavin T (ThT), ThT analogs, Congo Red dye, carbazole derivatives, perylene, amyloid-binding fluorescent peptides, conformational specific probes, antiamyloid antibodies-based immunochemoluminiscence and any of the compounds and strategies defined in Aliyan A., Cook N.P. , and Angel A. Martí, A:A. Interrogating Amyloid Aggregates using Fluorescent Probes Chemical Reviews 2019 119(23), 11819-11856.DOI: 10.1021/acs.chemrev.9b00404).

In a preferred embodiment, alone or in combination with other preferred embodiments of the invention, the amyloid-specific probe is selected from the list consisting of thioflavin T (ThT), ThT analogs, Congo Red dye, carbazole derivatives, perylene, amyloid-binding fluorescent peptides, conformational specific probes and antiamyloid antibodies-based immunochemoluminiscence. Amyloid-specific probes and/or strategies defined in Aliyan A., Cook N.P, and Angel A. Martí, A.A. Interrogating Amyloid Aggregates using Fluorescent Probes Chemical Reviews 2019 119 (23), 11819-11856.DOI: 10.1021/acs.chemrev.9b00404) can also be used in a preferred embodiment.

In a preferred embodiment, alone or in combination with other preferred embodiments of the invention, the amyloid-specific probe is fluorescent, giving a fluorescent signal. More preferably, the amyloid-specific probe is ThT.

In a preferred embodiment of the step d), alone or in combination with the rest of preferred embodiments, the incubation with agitation is carried out during at least 24h. More preferably, during at least 30h.

In another preferred embodiment of the step d), alone or in combination with the rest of preferred embodiments, the agitation is carried out by orbital shaking and/or centrifugation in at least 100 rpm, more preferably at least, 300 rpm. In another preferred embodiment of the step d), alone or in combination with the rest of preferred embodiments, the agitation is carried out by orbital shaking and/or centrifugation at less than 900 rpm, more preferably between 300 and 900 rpm.

In another preferred embodiment of the step d), alone or in combination with the rest of preferred embodiments, the incubation with agitation is carried out in presence of glass beads.

In another preferred embodiment of the step d), alone or in combination with the rest of preferred embodiments, the incubation with agitation is carried out at a temperature ranging from 20 to 55 ºC (including the end values of the range), preferably at 37 ºC.

In another preferred embodiment of the amplification-based screening method of the invention, alone or in combination with the rest of preferred embodiments, the reaction mixture prepared in step (a) and the reaction mixture prepared in step (b), further comprise GdnHCl (Guanidinium chloride or guanidine hydrochloride) or urea. More preferably, they comprise GdnHCl or urea at a concentration ranging from 0.4 to 0.6 M (including the end values of the range).

Additionally, the reaction mixture prepared in step a) and the reaction mixture prepared in step b), can also comprise other buffers/reagents known in the state of the art. In a preferred embodiment, alone or in combination with the rest of preferred embodiments, the reaction mixture prepared in step a) and the reaction mixture prepared in step b), further comprise Tris-HCl, preferably Tris-HCl pH 7.5 at a concentration of 10 mM, NaCl, preferably at a concentration of 150 mM, and dithiothreitol (DTT), preferably at a concentration of 1 mM.

Any of the polypeptides of the invention may be added in the step c) of the amplification-based screening method of the invention. Polypeptides of the invention have been defined in the first aspect of the invention, and their definitions as well as their preferred embodiments apply to the present aspect of the invention. In a preferred embodiment, the polypeptide of the invention added in the step c) is the MetO-PLD polypeptide of the invention with a concentration in the reaction mixture ranging from 5 to 15 $\mu$M (including the end values of the range). More preferably, the polypeptide of the invention added in the step c) is the MetO-PLD polypeptide of the invention with a concentration selected from the list consisting of 5, 6, 7, 8, 9, 10, 11, 12, 13, 14 and 15 $\mu$M.

As mentioned above, the biological sample is a previously isolated biological sample from a biological system. The term "biological system" can be defined as any biological system that would be understood by a skilled person to provide insight as to the effects a compound may have on TDP-43 aggregation.

The system may comprise: (i) an experimental test subject when an *in vivo* test is to be employed; in the case the experimental test subject is a human being, the present screening method only considers an *in vivo test* under strictly clinical trial conditions with the informed consent of the patient concerned; (ii) a biological sample from a subject suffering a TDP-43 proteinopathy; (iii) a cell line model (e.g. a cell expressing TDP-43 or a cell engineered to express the protein); or even (iv) an *in vitro* system that comprises TDP-43 and simulates the physiological environment such that TDP-43 aggregation can be assessed.

In other preferred embodiment, alone or in combination with the rest of preferred embodiments, the biological system is an animal suffering a TDP-43 proteinopathy.

In other preferred embodiment, alone or in combination with the rest of preferred embodiments, the biological system is a TDP-43 transgenic animal.

In a preferred embodiment, alone or in combination with the rest of preferred embodiments, the biological sample is an isolated biological sample from a subject suffering a TDP-43 proteinopathy. More preferably, the isolated biological sample is selected from the list consisting of a cerebrospinal fluid (CSF), a serum, a spinal-cord, a muscle sample and a cortex sample from a subject suffering a TDP-43 proteinopathy.

In a preferred embodiment of the amplification-based screening method of the invention, alone or in combination with the rest of preferred embodiments, the TDP-43 proteinopathy is amyotrophic lateral sclerosis (ALS).

In another preferred embodiment of the amplification-based screening method of the invention, alone or in combination with the rest of preferred embodiments, the TDP-43 proteinopathy is frontotemporal lobar degeneration (FTLD).

Additionally, the measuring of the signals in step e) can be carried out at different times. In a preferred embodiment, alone or in combination with the rest of preferred embodiments, the step e) comprises measuring the signals at different times, wherein said different times are at least two different times. More preferably, at 3, 4, 5, 6, 7 8, 9, 10 or more different times.

Besides, the measuring of the signals in step e) can be carried out during a continuous period of time. Thus, in other more preferred embodiment, the step e) comprises measuring the signals during a period of time of at least 24 h, even more preferably during a period of time of at least 30 h.

In the present invention, in absence of treatment or when the test compound is not effective, TDP-43 aggregates in the biological system are formed and/or are not reduced, and MetO polypeptides of the invention amplify TDP-43 seeds. Subsequently, the intensity of the measured signal changes by comparison with a control, wherein not a treatment with the mentioned compound has been previously produced. Said change can be a decrease of the intensity of the signal by comparison with a control not contacted with the test compound or an increase of the time at which half of the maximum intensity is reached (also called in the present document as "t1/2") by comparison with a control not contacted with the test compound.

In a preferred embodiment of the amplification-based screening method of the invention, alone or in combination with the rest of preferred embodiments, the step f) of the method comprises selecting, as a prophylactic and/or therapeutic candidate compound for a TDP-43 proteinopathy, a test compound that changes the intensity of the signal wherein the change is a decrease of the intensity of the signal or an increase of t1/2 compared to the measured signal in the control reaction mixture (ii) (control condition, not contacted with the test compound).

In a more preferred embodiment, the change is a decrease of the intensity of the signal measured in the reaction mixture (i) by comparison with the control reaction mixture (ii) by a factor of at least 3, even more preferably, by a factor of, at least 3 produced at 30 h since the amplification step is carried out. In a still more preferred embodiment, the change is a decrease of the intensity of the signal measured in the reaction mixture (i) by comparison with the control reaction mixture (ii) by a factor of at least 10, even more preferably, by a factor of, at least 10 produced at 30 h since the amplification step is carried out.

In another more preferred embodiment, the change is an increase of t1/2 measured in the reaction mixture (i) by comparison with the control reaction mixture (ii) by a factor of at least 2.

In an even more preferred embodiment, when the amyloid-specific probe is ThT, the step f) of the screening amplification-based screening method of the invention comprises selecting, as a prophylactic and/or therapeutic candidate compound for a TDP-43 proteinopathy, a test compound that changes the intensity of the ThT fluorescence, wherein said change is a decrease of the ThT intensity fluorescence or an increase of t1/2 measured in the reaction mixture (i) by comparison with the control reaction mixture (ii).

In a more preferred embodiment, the change is a decrease of the fluorescence intensity of the signal measured in the reaction mixture (i) by comparison with the control reaction mixture (ii) by a factor of at least 3, even more preferably, by a factor of, at least 3 produced at 30 h since the amplification step is carried out. In a still more preferred embodiment, the change is a decrease of the fluorescence intensity of the signal measured in the reaction mixture (i) by comparison with the control reaction mixture (ii) by a factor of at least 10, even more preferably, by a factor of, at least 10 produced at 30 h since the amplification step is carried out.

In another more preferred embodiment, the change is an increase of t1/2 measured in the reaction mixture (i) by comparison with the control reaction mixture (ii) by a factor of at least 2.

*Other uses of the polypeptides of the invention*

Also based on the above-mentioned TDP-43 aggregates amplification properties of the MetO polypeptides of the invention, other uses have been developed as defined by the following aspects of the invention.

In other aspect, the invention provides the use of any of the MetO polypeptides of the invention for the *in vitro* amplification of pathological TDP-43 aggregates in a sample, hereinafter the "amplification use of the invention".

The term "sample" can be defined as part or small quantity of a thing which is considered to be representative of the whole and which is taken or separated from the thing which is considered representative of the whole and which is taken or separated from it for study, analysis or experimentation.

A preferred embodiment provides the amplification use of the invention in an isolated biological sample from a subject.

The term "subject", as used in the present document, refers to any animal, preferably a mammal, and includes, but is not limited to, domestic and farm animals, primates, and humans. In a preferred embodiment, alone or in combination with the rest of preferred embodiments, the subject is a human being.

The term "isolated biological sample" refers to any sample isolated from a subject and includes, but is not limited to, biological fluids from an individual, obtained by any method known to a person skilled in the art for that purpose. Preferably, the isolated biological sample is selected from the list consisting of a cerebrospinal fluid sample, a serum sample, a spinal-cord sample, a cortex sample, a muscle sample, and any combination thereof.

In some embodiments of the amplification uses of the invention, the *in vitro* amplification is carried out in presence of GdnHCl (Guanidinium chloride or guanidine hydrochloride) or urea. More preferably, GdnHCl or urea at a concentration ranging from 0.4 to 0.6 M.

Moreover, based on the above-mentioned amplification properties of the MetO polypeptides of the invention of TDP-43 aggregates, they can be used for detection purposes.

Thus, other aspect of the invention relates to the use of any of the MetO polypeptides of the invention as a detection reagent for imaging TDP-43 aggregates, hereinafter the "detection reagent use of the invention"

The term "detection reagent", as used herein, refers to a substance able to detect, identify and/or determine another substance, in the present use by the amplification of TDP-43 propagons.

A preferred embodiment provides the detection reagent use of the invention in an isolated biological sample from a subject.

The terms "subject", "sample" and "isolated biological sample" have been previously described, and its definitions, as well as their preferred embodiments, apply to the present aspect of the invention.

*In vitro diagnostic method of the invention*

Based on the above-mentioned amplification properties of the MetO polypeptides of the invention of TDP-43 propagons, they can also be used for *in vitro* diagnostic purposes.

It is usually difficult to make an early diagnosis of ALS. In fact, the time lag from symptom onset to definitive diagnosis is usually 18 months, which is a critical delay for effective treatment in any clinical trial, being imperative to find efficient biomarkers for early diagnosis and monitoring of the disease.

Other aspect of the present invention relates to a method for the *in vitro* diagnosis of a TDP-43 proteinopathy in an isolated biological sample from a subject, hereinafter the "diagnostic method of the invention", which comprises the following steps:

a) preparing a reaction mixture, wherein said reaction mixture comprises the isolated biological sample, preferably wherein the isolated biological sample is selected from the list consisting of a cerebrospinal fluid sample, a serum, a spinal-cord sample, a muscle sample and a cortex sample, and an amyloid-specific probe, wherein said probe is able to produce a detectable signal;

b) adding any of the polypeptides of the invention to the reaction mixture prepared in step a);

c) an amplification step, which comprises incubating with agitation the reaction mixture obtained in step b), preferably, wherein said agitation is carried out in presence of beads;

d) measuring, simultaneously to when the amplification step c) is performed), the detectable signal;

e) comparing the detectable signal with reference values, wherein an intensity increase in the measured detectable signal and/or a decrease of the time at which half of the maximum intensity of the signal is reached (decrease of $t_{1/2}$), compared to the reference value, indicates that the subject has pre-formed TDP-43 propagons and, therefore, a TDP-43 proteinopathy.

The phrase "simultaneously to when the amplification step c) is performed", as used herein, means that the step d) is performed at the same time that step c).

Examples of "amyloid-specific probes", as well as related preferred embodiments have been previously described, applying equally to the diagnostic method of the invention.

In a preferred embodiment, alone or in combination with the rest of preferred embodiments, the incubation is carried out during at least 24h. More preferably, during at least 30h.

In another preferred embodiment of the amplification-based screening method of the invention, alone or in combination with the rest of preferred embodiments, the reaction mixture prepared in step (a) further comprises GdnHCl (Guanidinium chloride or guanidine hydrochloride) or urea. More preferably, it comprises GdnHCl at a concentration ranging from 0.4 to 0.6 M (including the end values of the range).

Additionally, the reaction mixture prepared in step a) can also comprise other buffers/reagents known in the state of the art. In a preferred embodiment, alone or in combination with the rest of preferred embodiments, the reaction mixture further comprises Tris-HCl, preferably Tris-HCl pH 7.5 at a concentration of 10 mM, NaCl, preferably at a concentration of 150 mM, and dithiothreitol (DTT), preferably at a concentration of 1 mM.

Any of the polypeptides of the invention may be added in the step b) of the diagnostic method of the invention. Polypeptides of the invention have been defined in the first aspect of the invention, and their definitions as well as their preferred embodiments apply to the present aspect of the invention. In a preferred embodiment, the polypeptide of the invention added in the step b) is the MetO-PLD polypeptide of the invention with a concentration in the reaction mixture ranging from 5 to 15 $\mu$M (including the end values of the range). More preferably, the polypeptide of the invention added in the step b) is the MetO-PLD polypeptide of the invention with a concentration selected from the list consisting of 5, 6, 7, 8, 9, 10, 11, 12, 13, 14 and 15 $\mu$M.

In a preferred embodiment, the amyloid-specific probe is fluorescent, more preferably ThT.

In a preferred embodiment of the step c), alone or in combination with the rest of preferred embodiments, the incubation with agitation is carried out during at least 24h. More preferably, during at least 30h.

In another preferred embodiment of the step c), alone or in combination with the rest of preferred embodiments, the agitation is carried out by orbital shaking and/or centrifugation in at least 100 rpm, more preferably at least, 300 rpm. In another another preferred embodiment of the step d), alone or in combination with the rest of preferred embodiments, the agitation is carried out by orbital shaking and/or centrifugation at less than 900 rpm, more preferably between 300 and 900 rpm.

In another preferred embodiment of the step c), alone or in combination with the rest of preferred embodiments, the incubation with agitation is carried out in presence of glass beads.

In another preferred embodiment of the step c), alone or in combination with the rest of preferred embodiments, the incubation with agitation is carried out at a temperature ranging from 20 to 55 ºC (including the end values of the range), preferably at 37 ºC.

The term "TDP-43 proteinopathy" has been previously described, and its definition, as well as its preferred embodiments, apply to the diagnostic method of the invention. The term "subject", has been previously described, and its definition, as well as its preferred embodiments, apply to the diagnostic method of the invention.

In a preferred embodiment, the TDP-43 proteinopathy is amyotrophic lateral sclerosis (ALS).

In other preferred embodiment, the TDP-43 proteinopathy is frontotemporal lobar degeneration (FTLD).

Additionally, the measuring of the signals in step d) can be carried out at different times. In a preferred embodiment, alone or in combination with the rest of preferred embodiments, the step d) comprises measuring the signals at different times, wherein said different times are at least two different times. More preferably, at 3, 4, 5, 6, 7 8, 9, 10 or more different times.

Besides, the measuring of the signals in step d) can be carried out during a continuous period of time. Thus, in other more preferred embodiment, the step d) comprises measuring the signals during a period of time of at least 24 h, even more preferably during a period of time of at least 30 h.

The term "reference values" as used herein refers to a signal measured, preferably ThT fluorescence measured, in a biological sample isolated from a healthy subject, a biological sample isolated from a subject non-suffering a TDP-43 proteinopathy, and/or in a sample without TDP-43 aggregates. Preferably, the reference value is a signal measured in a biological sample isolated from a subject non-suffering a TDP-43 proteinopathy. more preferably wherein said isolated biological sample is selected from the list consisting of a cerebrospinal fluid sample, a serum sample, a spinal-cord sample, a cortex sample and a muscle sample.

In the present invention, an intensity increase in the measured detectable signal and/or a decrease of the time at which half of the maximum intensity of the signal is reached (decrease of t1/2), compared to the reference value, indicates that the subject has pre-formed TDP-43 propagons and, therefore, a TDP-43 proteinopathy, given that the MetO polypeptide of the invention amplifies TDP-43 propagons.

In a preferred embodiment, alone or in combination with the rest of preferred embodiments, at least a 3-fold increase of the intensity in the in the measured ThT fluorescence and/or at least a 3 h of decrease of the time at which half of the maximum intensity is reached (decrease of t1/2), compared to the reference value, indicates that the subject has pre-formed TDP-43 aggregates and, therefore, a TDP-43 proteinopathy.

In this embodiment, at least a 3-fold increase in the measured ThT fluorescence and/or at least a 3 h of decrease of the time at which half of the maximum intensity is reached (decrease of t1/2), compared to the reference value, indicates that the subject has pre-formed TDP-43 aggregates and, therefore, a TDP-43 proteinopathy.

In a more preferred embodiment, when the amyloid-specific probe is ThT, at least a 3-fold increase of the intensity in the measured ThT fluorescence and/or at least a 3 h of decrease of the time at which half of the maximum intensity is reached (decrease of t1/2), compared to the reference value, indicates that the subject has pre-formed TDP-43 aggregates and, therefore, a TDP-43 proteinopathy.

The terms "subject", "sample" and "isolated biological sample" have been previously described, and its definitions, as well as their preferred embodiments, apply to the present aspect of the invention.

Terms used to define the methods of the invention have been previously described, and its definitions, as well as their preferred embodiments, apply to the present aspect of the invention.

## DESCRIPTION OF THE DRAWINGS

**Fig. 1. CNBr resistant PLD fragments in ALS spinal cord samples.** Western blot detection of modified TDP-43 fragments treated in the absence (-) and presence (+) of CNBr developed using an anti-phospho Ser410 antibody. Samples were obtained from spinal cord of control (C) and ALS patients, and TDP-43 aggregates purified following the SarkoSpin protocol (Perez-Berlanga et al. 2019 Bio Protoc 9 (22): e3424).

**Fig. 2.** ThT-binding kinetics of 9 $\mu$M MetO PLD (A,B) in the presence of CSF from control and two ALS patients (CSF-ALS1 and CSF-ALS2, respectively). Reactions were performed in either 0.4M GdnHCl (A) or 0.5M urea (B).

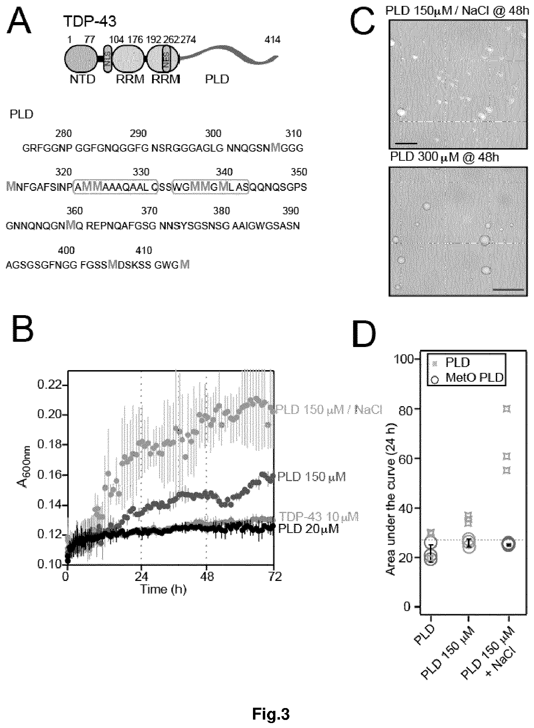

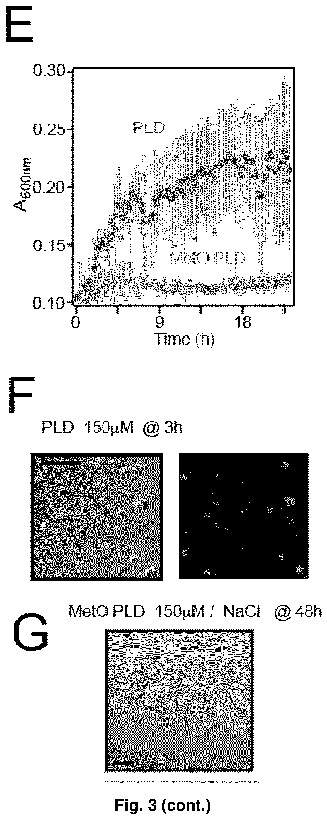

**Fig. 3. Methionine sulfoxidation impairs TDP-43's PLD phase separation. A)** Cartoon representation of the domain architecture of TDP-43 (top). The disordered PLD (region 274-414 relative to TDP-43) is represented by a line. PLD primary structure, with the Met residues highlighted in bold (bottom). The double $\alpha$-helices are limited by grey boxes. **B)** Turbidity measurements showing concentration- and salt-dependent Liquid-Liquid Phase Separation (LLPS) for the PLD. NaCl refers to the presence of 150 mM NaCl. **C)** Differential Interference Contrast (DIC) microscopy images showing condensates formed by the PLD in the specified conditions. Scale bars correspond to 25 $\mu$m. NaCl refers to the presence of 150 mM NaCl. **D)** LLPS of MetO PLD in the corresponding samples (circles), compared to unmodified PLD (stars), as measured by the area under the turbidity curves after 24h of incubation. Gray broken line corresponds to the averaged LLPS of unmodified PLD. NaCl refers to the presence of 150 mM NaCl. **E)** Turbidity measurements showing that methionine sulfoxidation impairs phase separation. Data shown corresponds to 300 $\mu$M unmodified PLD (dark grey) and 300 $\mu$M MetO PLD (light grey). **F)** DIC (left) and fluorescence (right) microscopy images of the condensates formed by 150 $\mu$M PLD after 3h of incubation at 25 °C. The scale bar corresponds to 25 $\mu$m. **G)** DIC image of 150 $\mu$M MetO PLD in the presence of 150 mM NaCl after 48 h of incubation at 25 °C showing the absence of condensates. The scale bar corresponds to 25 $\mu$m. Unless otherwise stated, samples contained 150 mM KCl.

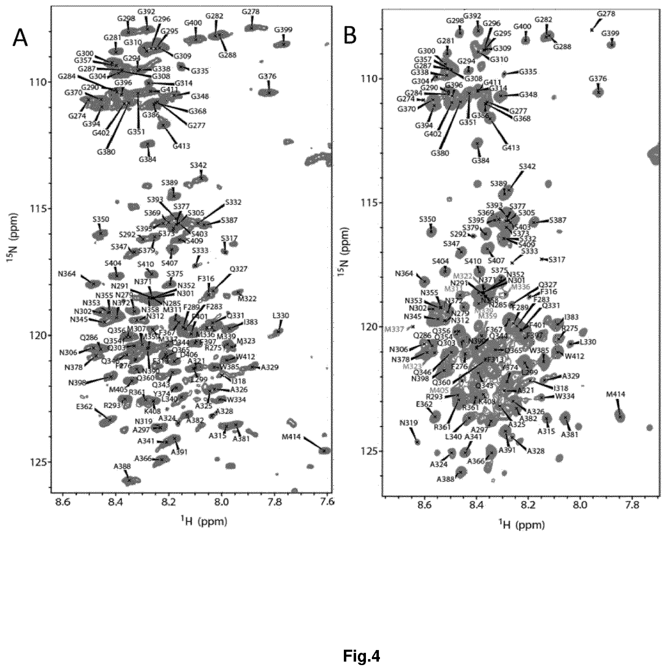

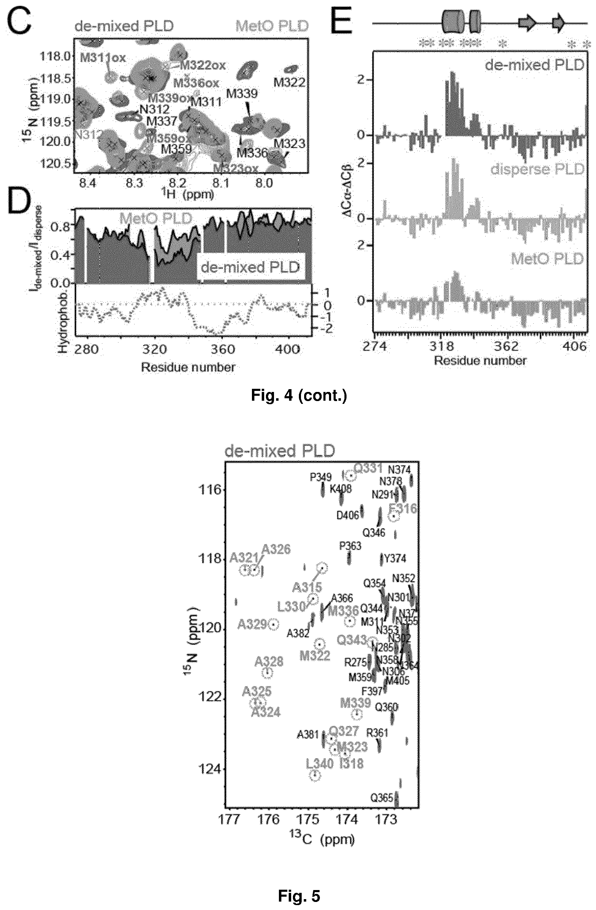

**Fig. 4. Methionine sulfoxidation reshapes TDP-43's PLD structure.** Assigned $^{15}$N-HSQC spectrum of **(A)** de-mixed unmodified PLD and **(B)** disperse MetO PLD. Spectra were obtained at 300 $\mu$M protein concentrations and sulfoxidized methionines are highlighted in gray. **(C)** Detailed region of the overlay of the $^{15}$N-HSQC spectra from de-mixed unmodified PLD (dark gray) and MetO PLD (light gray) showing the large shifts for the Met moieties upon methionine sulfoxidation. MetO crosspeaks are highlighted in bold. **(D)** Comparison of the NMR signal intensity of unmodified PLD (dark gray) and MetO PLD (gray) upon de-mixing shows a reduced broadening (reduced Intensity) in the region 305-345 for MetO PLD. The preceding region (280-305) is also partially broadened in both proteins. The plot at the bottom shows the hydrophobicity of the PLD. **(E)** Secondary chemical shifts ($\Delta C\alpha$-$\Delta C\beta$) analysis for 300 $\mu$M unmodified PLD (top), 25 $\mu$M unmodified PLD (middle) and 300 $\mu$M MetO PLD (bottom). In these plots, positive values indicate acquisition of $\alpha$-helical conformations, while negative values correspond to $\beta$-strand structures. The schematic cartoon at the top highlights the two $\alpha$-helices (in cylinders) and $\beta$-strands (arrows) formed in the PLD. Met residues are located with asterisks. For clarity, Met residues were removed from the MetO PLD plot (bottom) due to the strong shifts upon oxidation.

**Fig. 5. Selected region of the CON spectrum of de-mixed PLD.** The spectrum of a 300 $\mu$M sample of de-mixed unmodified PLD shows the absence of crosspeaks corresponding to the region 305-345. Assignments are shown in black, while the expected location of the missing peaks are labeled in circles.

**Fig. 6. MetO PLD undergoes a significant conformational rearrangement.** Circular dichroism (CD) spectra of unmodified PLD (dark grey) and MetO PLD (light grey), showing a strong shift towards disordered conformations upon methionine sulfoxidation.

**Fig. 7. MetO PLD undergoes different fibrillary aggregation compared to PLD. (A)** Amyloid aggregation kinetics as measured by ThT fluorescence. Samples contained either 150 mM KCl or 150 mM NaCl, as noted. **(B)** ThT aggregation kinetics for 20, 12 and 10 $\mu$M PLD and for 10 $\mu$M PLD in presence of increasing amounts of PLD seeds. **(C)** ThT aggregation kinetics for 20, 12 and 10 $\mu$M MetO PLD and for 10 $\mu$M MetO PLD in presence of increasing amounts of MetO PLD seeds. Errors are lower than 0.35 in **(B)** and 0.25 in **(C)** in normalized fluorescence and are not included for simplicity. **(D)** Logarithmic plot of the time at which half of the monomer is converted to fibril, $t_{1/2}$, versus the initial monomer concentration. The scaling exponent largely differs between MetO PLD (-0.66) and PLD (-0.09), suggesting that the aggregation is governed by distinct mechanisms.

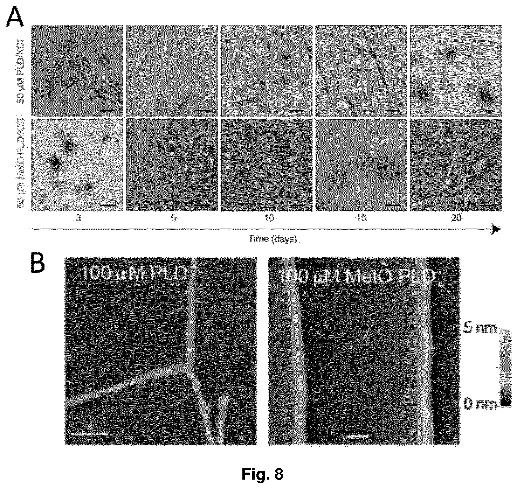

**Fig. 8. Microscopy imaging of PLD and MetO PLD amyloid aggregates. (A)** Characterization of PLD and MetO PLD amyloid formation by TEM. Representative Images obtained from 50 $\mu$M PLD and 50 $\mu$M MetO PLD samples aged at the indicated times. MetO PLD forms fibrils at a significantly slower rate, and the fibrils formed are morphologically different. The scale bars represent 200 nm. **(B)** Atomic force microscopy topographic characterization of the fibrils formed by 100 $\mu$M PLD (left) and 100 $\mu$M MetO PLD (right) samples aged for 4 months. PLD fibrils show variable diameter along its length. Scale bars correspond to 400 nm. Height scale is 5 nm.

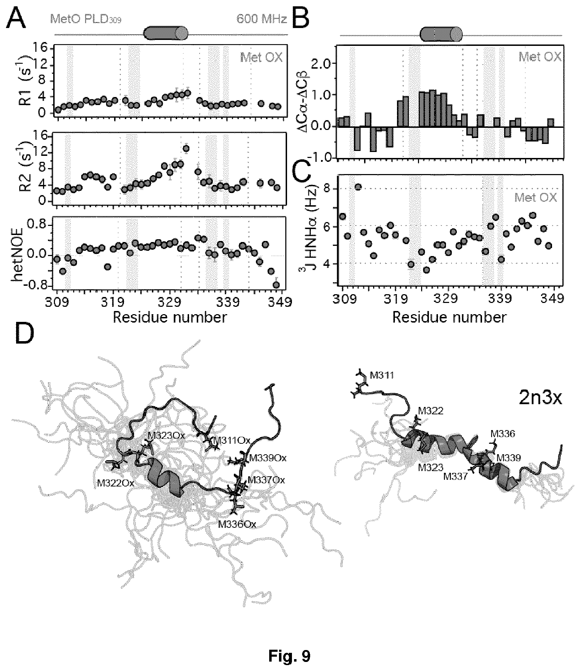

**Fig. 9. Methionine sulfoxidation promotes significant disorder in the PLD. (A)** Longitudinal (R1, top), transverse (R2, middle) rate constants and heteronuclear ($^1$H)-$^{15}$N NOE (bottom) relaxation parameters obtained for MetO PLD$_{309}$ at 600 MHz. The magnitude of all relaxation parameters indicates that the protein is highly dynamic. **(B)** Secondary chemical shifts ($\Delta$C$\alpha$-$\Delta$C$\beta$) for MetO PLD$_{309}$. **(C)** J-coupling assessment of the secondary structure propensities for MetO PLD$_{309}$. Values around 4 Hz indicate $\alpha$-helix formation, while values around 8 Hz are typical of $\beta$-strand structures. **(D)** Structural ensemble of the 20 conformers of MetO PLD$_{309}$ with the lowest conformational energy as calculated by CYANA (left), compared to the ensemble of 10 conformers of a comparable PLD fragment (PDB code 2n3x, right). The lowest energy structure is displayed on top of the corresponding ensemble, with the rest of the conformers shown in transparent representation. Structures were aligned onto the structured elements. Met residues are highlighted in sticks representation. Gray broken lines in A-B plots limit the two $\alpha$-helices present in the PLD, while gray bars mark the location of Met residues. Diagrams on top of the plots (A-B) represent the $\alpha$-helix formed in MetO PLD$_{309}$ (gray cylinder).

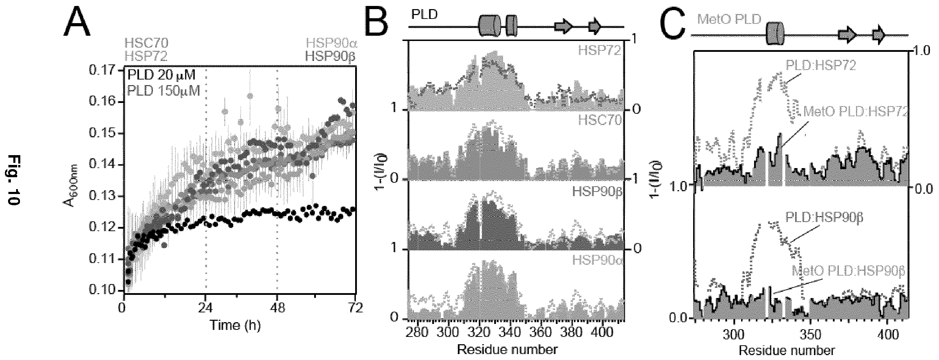

**Fig. 10. The interplay with chaperones is prevented in MetO PLD given the distortion of the double $\alpha$-helix motif. (A)** Turbidity measurements of the PLD in presence of the indicated chaperones (all in 1:2 molar ratios). Error bars are not included in the PLD plot (black dots) for clarity. **(B)** NMR signal intensity plots of the PLD in complex with the indicated chaperones (all in a 1:2 molar ratio). For comparison, the plots are overlaid with the data corresponding to de-mixed PLD (broken gray line) and HSP70 (light gray broken line). **(C)** NMR intensity plots for MetO PLD in presence of the chaperones (gray bars) in comparison to the unmodified PLD:chaperone interactions in identical molar ratios (broken lines).

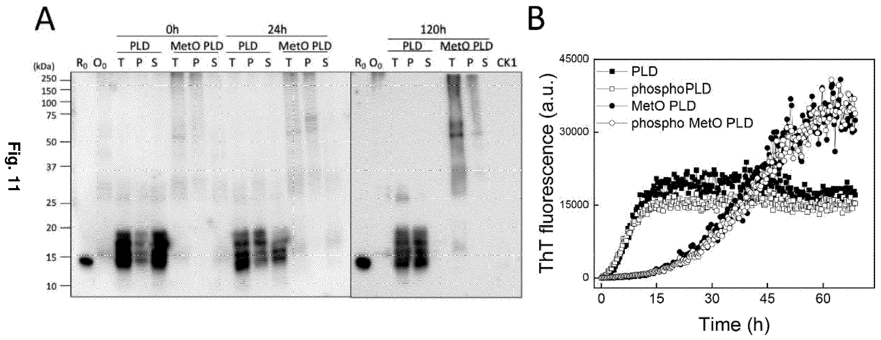

**Fig. 11. MetO modifies CK1$\delta$ phosphorylation pattern. (A)** 20 $\mu$M PLD and MetO PLD were incubated at 37 °C and 0.4 $\mu$M CK1$\delta$ was added at the indicated points and left for 1 hour at 37 °C. The immunoblot was developed using anti-phospho Ser410 antibody. $R_0$ and $O_0$ samples refer to unmodified (R) and MetO PLD (O) samples which were incubated in the absence of CK1$\delta$, $T$ indicates total fraction, P indicates pellet and S soluble fractions. Phosphorylated monomers are resolved as discrete bands of 15-19 kDa, whereas phosphorylated aggregates run as a 25-250 kDa smear. **(B)** Kinetics of amyloid fibril formation as measured by ThT fluorescence for 20 $\mu$M PLD samples.

**Examples**

**1. Materials and methods**

**1.1. Human samples**

Cerebrospinal fluid (CSF) and spinal-cord samples for control and two ALS-patient donors were provided by the Banco de Tejidos of the Fundacion Cien (BTFCIEN) and their analysis was performed in the institute de Salud Carlos III (ISCIII) in collaboration with Dr. M. Calero.

**1.2. CNBr (cyanogen bromide) digestion**

Human tissue samples were processed following SarkoSpin methodology (Perez-Berlanga et al. 2019 Bio Protoc 9 (22): e3424) to isolate insoluble aggregates. Pellets containing aggregated TDP-43 were dissolved in 8 M urea and 70% formic acid. 3 mg of CNBr (Merck) per mg of protein were added and the reaction triggered under inert atmosphere and in the absence of light for 48 h at room temperature. Formic acid was removed in a speed-vac. Samples were resuspended in Laemmli buffer and submitted to western blotting using rabbit monoclonal anti-phospho Ser410 TDP-43 antibody (Sigma-Aldrich, diluted 1:1000 in blocking buffer).

**1.3. Seed/Propagon amplification assay**

For CSF TDP-43 RT-QuIC seed detection, 5 $\mu$L of each CSF sample was added to 145 $\mu$L of reaction mix in clear-bottom black 96-well microplates containing a 3 mm glass bead (Sigma). The final composition of the reaction mix was 10 mM Tris-HCl pH 7.5, 150 mM NaCl, 0.5 mM EDTA, 1 mM DTT, protease inhibitors (1 Complete tablet/10 ml), 0.4M GdnHCl or 0.5M urea, and 9 $\mu$M MetO PLD (the MetO-PLD polypeptide of the invention) as a substrate. Substrates prepared in 8M urea or 6M GdnHCl were defrost at room temperature and filtrated using a 100 kDa-pore size microcon to remove pre-formed aggregates before its use in the reaction. After sealing, the plate was incubated in a FLUOstar OMEGA reader (BMG Labtech, Germany) at 37°C using a 300 rpm double orbital agitation over a period of 50 h as previously described (Martinez et al. 2011 PLoS One 6, e27999).

**1.4. Protein sample preparation**

Both full-length human TDP-43 (UniProt KB Q13148) and its fragment 274-414 (PLD) were subcloned in modified pET28a vectors (Novagen) containing Thioredoxin (TXA) as a fusion protein followed by a six-histidine tag for Ni$^{2+}$ affinity purification and a Tobacco Etch Virus (TEV) protease cleavage site. All cloning procedures were performed following the Gibson Assembly method (New England Biolabs). The PLD fragment was used as template for the generation and cloning the fragment 309-350 of TDP-43 (PLD$_{309}$) using the same plasmid platform. Human-chaperone (HSC70, HSP72, HSP90$\alpha$ and HSP90$\beta$)-cDNA sequences were cloned into respective pET28a vectors (GenScript). All sequences were verified by DNA sequencing. Cloning procedures were performed with the following oligonucleotides in both DH5$\alpha$ and XL1 *E.* coli strains:

| | Forward | Reverse |
|---|---|---|
| **TDP-43 insert** | 5´ATTTCCAGGGATCCATGTCTGAAT ATATTCG3´ (SEQ ID NO: 4) | 5´GTGGTGCTCGAGTTACATTCCCCA GCCAGAAG3´(SEQ ID NO: 5) |
| **TDP-43 vector** | 5 'CTCGAGCACCACCACCACCAC3' (SEQ ID NO: 6) | 5´GACATGGATCCCTGGAAATACAGG TTTTC3´(SEQ ID NO: 7) |
| **PLD insert** | 5´ATTTCCAGGGATCCGGAAGATTTG GTGGTAATC3´(SEQ ID NO: 8) | 5´GTGGTGCTCGAGTTACATTCCCCA GCCAGAAG3´(SEQ ID NO: 9) |
| **PLD vector** | 5'CTCGAGCACCACCACCACCAC3' (SEQ ID NO: 10) | 5´CTTCCGGATCCCTGGAAATACAGG TTTTC3´(SEQ ID NO: 11) |

(continued)

| | Forward | Reverse |
|---|---|---|
| PLD$_{309}$ vector | 5 'CTCGAGCACCACCACCACCAC3' (SEQ ID NO: 12) | 5′ GAAAACCTGTATTTCCAGGGATCCG GTGG3′(SEQ ID NO: 13) |
| PLD$_{309}$ insert | 5′ ATTTCCAGGGATCCGGTGGGATGAA CTTTG 3′(SEQ ID NO: 14) | 5′ CAGTCAGGCCCATCGTAACTCGAGC ACC 3′(SEQ ID NO: 15) |

TDP-43, PLD, and PLD$_{309}$ fragments were produced in Rosetta 2 (DE3) *E. coli* strain, inducing with 0.5 mM isopropyl β-d-1-thiogalactopyranoside (IPTG) at OD$^{600}$ - 0.7 at 25 °C for 20 h. For $^{15}$N/$^{13}$C isotopic labelling, M9 minimal media was used including 4g/l of $^{13}$C-glucose and 1 g/l of $^{15}$NH$^4$Cl as the sole sources of C and N, respectively.

Cells were harvested by a 20 min centrifugation at 5,300 g and resuspended in 30 ml/L of lysis buffer (20 mM Tris-HCl/500 mM NaCl/10 mM imidazole [pH 8]), including 5 μl of protease inhibitors (ThermoFisher Scientific), 0.08 mg/ml of DNAse I and RNAse A (both from Sigma-Aldrich) and 1 mg/ml lysozyme (Sigma-Aldrich) and sonicated. After centrifugation at 15,500 g for 20 min, the pellet was resuspended in the aforementioned buffer and incubated for 15 min at 37 °C and harvested by centrifugation. Pellets were dissolved in 20 mM Tris-HCl/500 mM NaCl/8 M urea/10 mM imidazole [pH 8], clarified by centrifugation at 15,500 g during 20 min and loaded onto pre-equilibrated Ni2+ affinity columns (ABT), adding 250 mM imidazole to the buffer for the elution. TEV digestion was performed in 20 mM Tris-HCl/150 mM NaCl/1.5 M urea [pH 8] buffer (TEV buffer), by an overnight incubation at 25 °C with 0.5-1 mg/ml of TEV protein (produced in house) per 15 mg of fusion protein. Cleaved TDP-43 and PLD proteins were directly cleared with a Ni2+ affinity purification as previously detailed. Buffers for the purification and cleavage of the PLD C415 variant were supplemented with 10 mM DTT. After purification, protein samples were concentrated using an ultrafiltration Merck AmiconTM bioseparation stirring cell with 10 kDa filter with a continuous Argon flow. Protein samples were snap-frozen at -80°C in small aliquots at concentrations around 200 μM. For PLD$_{309}$, cleaved proteins were purified by Ni$^{2+}$ affinity in the absence of urea, dialyzed into NMR buffer (described below), lyophilized and stored at -20 °C.

Thawed protein aliquots were clarified by centrifugation at 15,000 g for 15 min, followed by filtration through a 100 kDa filter (Microcon) at 10,000 g to remove preformed aggregates. When required, buffer exchange into 20 mM Hepes/10 mM KCl/0.03% sodium azide [pH 6.8] (LIF buffer); 20 mM Hepes/10 mM KCl/5 mM MgCl2/1 mM ATP/1 mM DTT/0.2 mM phenylmethylsulfonyl fluoride (PMSF)/0.03% sodium azide [pH 6.8] (NMR buffer) and 20 mM Hepes/6M GdnHCl [pH 6.8] was performed using Zeba desalting columns (ThermoFisher), followed by additional clarification steps at 15,000 g to remove aggregates. If needed, a concentration step using Amicon concentrators with 10 kDa and 3 kDa cutoff filters (Millipore) was performed. Protein concentration was determined spectrophotometrically using the protein's molar extinction coefficients (44920 and 17990 cm-1·M-1 for TDP-43 and PLD, respectively).

Molecular chaperones-were produced in Rosetta 2 (DE3) and BL21 (DE3) star *E. coli* strains, using 1 mM IPTG for induction at OD$^{600}$ - 0.8-0.9 during 4 h at 37 °C or 16 h at 25 °C. Cells were harvested and lysed as explained previously. Recombinant proteins in the soluble fraction were purified by Ni2+ affinity chromatography using high density NiSO$_4$ agarose beads (ABT), 20 mM Tris-HCl/500 mM NaCl/10 mM imidazole [pH 8] as binding buffer and 500 mM imidazole for elution. Proteins were further purified by size exclusion chromatography using HiLoad™ 26/60 Superdex™ 75 pg columns (Cytiva) in 10 mM Hepes/500 mM KCl/5 mM DTT [pH 7.5]. Fractions containing monomeric protein were pooled, concentrated using Vivaspin® 20 ultrafiltration tubes (Sartorius) and stored at -80 °C. Chaperone samples were buffer exchanged before the experiments into the abovementioned buffers using Zeba spin desalting columns (ThermoFisher).

### 1.5. PLD covalent modifications

To achieve methionine sulfoxidation (MetO), aliquots of PLD and PLD$_{309}$ were thawed or resuspended (for PLD$_{309}$) in the corresponding buffer and clarified at 15,000 g for 15 min prior to their incubation with 10 mM H$_2$O$_2$ at 25 °C for 16 h. Full MetO was validated by mass spectrometry and NMR spectroscopy. Excess H$_2$O$_2$ was removed by buffer exchange before the experiments using Zeba spin desalting columns (ThermoFisher).

Phosphorylation was achieved incubating 20-40 μM protein samples at 37 °C with 0.4 μM CK1δ (CK1δ residues 1-294, Sigma-Aldrich) in 20 mM Tris-HCl/150 mM NaCl/10 mM MgCl$_2$/10 mM β-mercaptoethanol/1 mM EGTA/2.5 mM ATP [pH 7]. Aliquots were collected at 0, 2, 24 and 120 h of incubation for immunoblotting. When required, duplicate samples were separated into soluble and pellet fractions using a 20 min centrifugation at 20,000 g before immunoblotting.

For NMR analysis, protein samples were incubated in 10 mM Hepes/150 mM NaCl/10 mM MgCl2/1 mM EGTA/2.5 mM ATP [pH 6.9] (phosphorylation buffer).

PLD C415 in LIF buffer was tagged with Alexa Fluor 488 C5 Maleimide (ThermoFisher Scientific) as indicated by the manufacturer. After tagging, 10 equivalents of iodoacetamide (Sigma-Aldrich) were added to prevent further dimer formation of untagged PLD C415. Excess of free dye was removed with Zeba desalting columns. A 70% labeling was confirmed spectrophotometrically.

### 1.6. Turbidimetry assays

PLD and MetO PLD samples prepared in LIF buffer ranging from 20-300 $\mu$M were incubated in 20 mM Hepes/150 mM KCl (or 150 mM NaCl where required)/4 mM ATP/4 mM DTT/0.2 mM PMSF/0.03% sodium azide [pH 6.8] (incubation buffer). 30 ng/$\mu$l of ethanol-precipitated yeast torula extract RNA (Sigma-Aldrich) was included in the incubation buffer. TDP-43 was used at the lower concentration of 10 $\mu$M due to its high aggregation propensity. Turbidity changes were measured at 25 °C monitoring the absorbance at 600 nm ($A^{600}$) every 15 min for 96 h, with 30 s of agitation (100 rpms) before each measurement in a multiwell plate reader FLUOstar Omega (BMG LABTECH) using 96-well flat-bottom plates (Porvair Sciences). ATP was refreshed every 24 h. All data were technically and biologically replicated and presented as the average between replicates with the standard deviations.

### 1.7. ThT binding kinetics

Protein samples ranging from 10-100 $\mu$M were incubated in 10 mM Hepes/150 mM KCl or NaCl/0.03% NaN$_3$ [pH 6.8], containing 10-15 $\mu$M ThT (Sigma-Aldrich) (aggregation buffer). Typically, 150 $\mu$l samples were placed in wells with a 3 mm glass ball and the kinetics of ThT binding was monitored by bottom reading of fluorescence intensity in a FLUOstar Omega microplate reader (BMG Labtech) at 37 ºC as previously described (Martinez J, et al. Selenomethionine incorporation into amyloid sequences regulates fibrillogenesis and toxicity. PloS One 6, e27999 (2011)). Measurements were performed using 450 nm excitation and 480 nm emission filters and 10 flashes reading every 15 min with 15 s of 200 rpm shaking before reading. All measurements were done in triplicate and the experiments were repeated at least twice using two different protein batches. For seeding experiments, the products of 120 h aggregation reactions were centrifuged 20 min at 20,000 g and the resulting pellets were resuspended in the aggregation buffer by a 15 min sonication at room temperature. The monomer concentration in seeds dispersions was determined by SDS-PAGE using freshly PLD and MetO PLD monomer samples as internal controls.

Kinetic measurements were carried out using 10 $\mu$M of protein monomer solutions containing the sonicated seeds at concentrations corresponding to 0, 0.1, 0.5 and 1 % of the monomer concentration. ThT binding to phosphoPLD was performed using 20 $\mu$M proteins and a 100:1 ratio of CK1$\delta$ in 20 mM Tris-HCl/150 mM NaCl/10 mM MgCl$_2$/10 mM $\beta$-ME/1 mM EGTA/2.5 mM ATP [pH 7.0].

### 1.8. DIC and Fluorescence microscopy

Differential Interference Contrast (DIC) and fluorescence microscopy images were obtained in a Leica AF6000 LX optical microscope and a Nikon Eclipse TE2000-U microscope, respectively. Protein samples prepared in both incubation and NMR buffers ranging from 20-300 $\mu$M were incubated at 25 °C for 48 h without agitation. Complex mixtures contained 20 $\mu$M PLD. For fluorescent microscopy, fluorescent-labeled PLD C415 was mixed with untagged PLD at a 1:500 molar ratio before buffer exchange. Samples were spotted onto glass slides (ThermoFisher scientific) and imaged upright.

### 1.9. NMR assignments, $^{15}$N $^{13}$C Relaxation analysis and Structure Calculation.

All the NMR spectra were acquired in an 800 MHz (1H) and a 600 MHz (1H) Bruker AVNEO spectrometers, both equipped with Z-gradient cryoprobes. 1H chemical shifts were referenced to the internal reference sodium trimethylsilylpropanesulfonate (DSS), and $^{15}$N and $^{13}$C chemical shifts were referenced indirectly to 1H using the corresponding gyromagnetic ratios (Markley JL, et al. Recommendations for the presentation of NMR structures of proteins and nucleic acids. IUPAC-IUBMB-IUPAB Inter-Union Task Group on the Standardization of Data Bases of Protein and Nucleic Acid Structures Determined by NMR Spectroscopy. J Biomol NMR 12, 1-23 (1998)). NMR assignments were obtained at 15 °C using 300 $\mu$M of 15N/13C-labeled PLD and MetO PLD in NMR buffer, where PLD is significantly de-mixed while MetO PLD remains largely disperse. These conditions differ substantially from those used previously. The following NMR spectra were acquired in the 800 MHz spectrometer to obtain unambiguous assignments: 2D 15N- and 13C-HSQCs, 2D CON, 2D CACO, 3D HNCO, HN(CA)CO, HNCA, CBCA(CO)NH, hCC(CO)NH (15 ms mixing time) and HBHA(CO)NH. Additional 3D NMR experiments were acquired on 25 $\mu$M PLD samples in NMR buffer to determine secondary chemical shifts at disperse and demixed PLD samples, respectively. MetO $PLD_{309}$ resonance assignments were obtained on the 600 MHz spectrometer at 15 °C on 500 $\mu$M samples in NMR buffer, and were based on the acquisition of the following 2D and 3D experiments: 2D 15N HSQC, 3D HNCO, HN(CA)CO, HNCA, HN(CO)CA, CBCA(CO)NH, HNCACB and hCC(CO)NH (15 ms mixing time), HNNH, HNHA and 15N-NOESY-HSQC and 13C-NOESY-HSQC (both using 120 ms mixing time). Assignments for MetO PLD and MetO $PLD_{309}$ were deposited in the BMRB (Biological Magnetic Resonance Bank) database (codes 51494 and 34737, respectively).

Loss of NMR signal intensity was calculated comparing the NMR signal intensities for de-mixed samples (for PLD) at 300 $\mu$M *vs.* the NMR signal intensity for disperse samples (at 25 $\mu$M). Secondary chemical shifts were calculated as $\Delta C\alpha - \Delta C\beta$, where $\Delta C\alpha$ and $\Delta C\beta$ are the differences between experimentally obtained C$\alpha$ and C$\beta$ chemical shifts at the specific protein concentrations and computed shifts for disordered PLD at the same temperature and pH (Kjaergaard M, Brander S, Poulsen FM. Random coil chemical shift for intrinsically disordered proteins: effects of temperature and pH. J Biomol NMR 49, 139-149 (2011)). In the MetO PLD/$PLD_{309}$ plots, secondary chemical shifts for Met residues were removed due to the strong C$\beta$ shifts upon sulfoxidation. $^3$JHNHA coupling constants were calculated on the basis of 3D HNHA experiments (Vuister GW, Delaglio F, Bax A. The use of 1JC alpha H alpha coupling constants as a probe for protein backbone conformation. J Biomol NMR 3, 67-80 (1993)). 15N longitudinal (R1), transverse (R2) and (1H)-$^{15}$N NOE relaxation data for 500 $\mu$M MetO $PLD_{309}$ in NMR buffer were obtained using standard Bruker pulse sequences acquired at 15 °C in the 600 MHz spectrometer. In all relaxation experiments the spectral width was 6579 Hz for 1H and 1277 Hz for $^{15}$N dimensions. Seven relaxation delays (20; 60; 150; 240; 460; 800 and 1600 ms) were used for $^{15}$N R1 measurements, while eight relaxation delays (16.96; 33.92; 67.84; 101.76; 152.64; 203.52; 356.16 and 695.36 ms) were used to measure $^{15}$N $R2$ values. For PLD and MetO PLD, $^{15}$N $R1$, $R2$ and (1H)-15N NOE relaxation data were acquired on 200 $\mu$M samples in NMR buffer at 15 °C in the 800 MHz spectrometer. In all relaxation experiments the spectral width was 9615 Hz for 1H and 1621 Hz for the $^{15}$N dimensions. Seven 15N $R1$ relaxation delays were used (100; 200; 300; 400; 600; 800 and 1000 ms) while eight relaxation delays (16.96; 33.92; 67.84; 135.68; 237.43; 339.19; 407.04 and 491.84 ms) were employed for 15N $R2$. Relaxation values and uncertainties were calculated by fitting an exponential decay to the data. Het-NOEs were calculated from the ratio of cross peak intensities in spectra collected with and without amide proton saturation during the recycle delay. Uncertainties in peak heights were determined from the standard deviation of the intensity distribution in signal-less spectral regions. $^{15}$N Carr Purcell Meiboom Gill (CPMG) relaxation dispersion experiments (Wang C, Grey MJ, Palmer AG. CPMG sequences with enhanced sensitivity to chemical exchange. J Biomol NMR 21, 361-366 (2001)) were used to obtain transverse relaxation rates ($^{15}$N$R2^{eff}$) as a consequence of the conformational exchange in the $\mu$s-ms timescale for 200 $\mu$M PLD and MetO PLD in NMR buffer at 15 °C. Each 15N CPMG relaxation dispersion experiment contained 9 interleaved $^{15}$N B1 fields: 0; 33; 133; 200; 267; 533; 733; 866 and 1000 Hz, with a total relaxation delay of 0.06 s in the 800 MHz spectrometer. $^{15}$N CPMG data was analyzed using Nessy (Bieri M, Gooley PR. Automated NMR relaxation dispersion data analysis using NESSY. BMC Bioinformatics 12, 421 (2011)), where 500 different Monte Carlo sampling simulations were performed using 7 independent fits to obtain the exchange rates (kex) and population of the assembled state (pB). Errors were obtained from:

$$R_2{}^{\mathrm{RMSD}} = \left\{ \sum \left( R_2{}^{i,exp} - R_2{}^{ave} \right)^2 / n \right\}^{1/2}$$

Where n is the number of frequencies, and $R2^{i,exp}$ and $R2^{ave}$ the individual experimental and averaged $R2$ values, respectively. *kex* values for PLD are reported as >2000 s$^{-1}$ to avoid under-interpretation of the CPMG data. The $S^2$ order parameter was derived from the chemical shifts. Sulfoxidized methionines were not included in the $S^2$ plots shown obtained by TALOS-N (Shen Y, Bax A. Protein backbone and sidechain torsion angles predicted from NMR chemical shifts using artificial neural networks. J Biomol NMR 56, 227-241 (2013)), to avoid over-interpretation of the order parameters due to the absence of sulfoxidized methionines in TALOS libraries. The overall correlation time ($\tau$c) was estimated from the ratios of the mean values of $T1$ and $72$ as $\tau$c $\simeq$ 1 / (4 $\pi *$ 15N frequency in Hz) $*$ ((6$* R2/R1$) - 7))½ which was derived from eqn. 8 of Kay *etal.* (Kay LE, Torchia DA, Bax A. Backbone dynamics of proteins as studied by 15N inverse detected heteronuclear NMR spectroscopy: application to staphylococcal nuclease. Biochemistry28, 8972-8979 (1989)) that considers J(0) and J($\omega$) spectral densities and discounts terms of higher frequencies from a subset of residues with little internal motion and no significant exchange broadening. This subset excluded residues with $T_2$ values lower than the average minus one standard deviation, unless their corresponding T1 values were larger than the average plus one standard deviation (Pawley NH, Wang C, Koide S, Nicholson LK. An improved method for distinguishing between anisotropic tumbling and chemical exchange in analysis of 15N relaxation parameters. J Biomol NMR 20, 149-165 (2001)). All NMR spectra were processed in Topspin 4.1.1 and analyzed in Sparky (Lee W, Tonelli M, Markley JL. NMRFAM-SPARKY: enhanced software for biomolecular NMR spectroscopy. Bioinformatics 31, 1325-1327 (2015)).

The NMR structure of MetO $PLD_{309}$ was calculated with the program CYANA v3.98.1316 based on experimental NOE-derived distance constraints and TalosN derived dihedral constraints (Shen Y, Bax A. Protein backbone and sidechain torsion angles predicted from NMR chemical shifts using artificial neural networks. J Biomol NMR 56, 227-241 (2013)) following the standard 7-cycle iterative process and a final annealing using the list of restraints obtained in the last cycle. Methionine sulfoxide residues were generated according to CYANA libraries. One hundred structures were generated using the mentioned procedure. Constraint files included 56 upper distance restraints for protons, complemented with 24 $\varphi$ and $\psi$ restrictions. The 20 conformers with the lowest target function values were selected and deposited in the Protein Data Bank under the accession number 8a6i. The structural ensembles were visualized and examined using MolMol (Koradi R, Billeter M, Wuthrich K. MOLMOL: a program for display and analysis of macromolecular structures. J Mol Graph 14, 51-55, 29-32 (1996)) and Pymol v2.0 (PyMOL Molecular Graphics System, Version 2.0 Schrodinger, LLC.). PROCHECK-NMR18 version 3.4.4 was used to analyze the quality of the refined structures. Statistics of the calculation are summarized as follows:

| | |
|---|---|
| NOE distance constraints: | |
| Intraresidual | 56 |
| Dihedral angle restraints | 24 |
| Stereospecific $^1$H assignments | 0 |
| Total | 80 |
| Maximal violation (Å) | 0.22 |
| CYANA target energy function (Å$^2$) | 0.05 $\pm$ 0.04 |
| RMSD (to mean coordinates): | |
| Heavy backbone (Å) (segment 324-332) | 0.41 |
| All heavy side chain atoms (Å) | 1.38 |
| Procheck Ramachandran plot statistics: | |
| Most favored regions (%) | 73.5 |
| Additionally allowed favored regions (%) | 26.5 |

NMR titrations were based on $^{15}$N-HSQC spectra measured at 15 °C in the 800 MHz spectrometer using 25-35 $\mu$M $^{15}$N/$^{13}$C-labeled PLD and MetO PLD in NMR buffer. Increasing amounts of unlabeled (therefore, NMR invisible) chaperones were added in the titrations. In these conditions, PLD phase separation is negligible. Fig. 10 show the data obtained at 1:2 molar ratios for PLD/MetO PLD:chaperones

**1.10. SDS-PAGE and Immunoblot**

Samples containing 10 $\mu$M protein prepared in Laemmli buffer with $\beta$-mercaptoethanol (BioRad) and heated at 95 ºC for 15 min were separated by SDS-PAGE using Mini-Protean® TGX Stain-FreeTM gels (#456-8126, BioRad). Electrotransference onto 0.2 $\mu$m immunoblot PVDF membranes was performed in Towbin buffer (25 mM Tris/192 mM Gly [pH 8.3] with 10% methanol) for 90 min at 4ºC. After blocking for 1 h in TBS (20 mM Tris-HCl/150 mM NaCl [pH 7.5]) containing 0.5% Tween 20 (TBS-T) and 0.5% bovine serum albumin (Sigma-Aldrich) at room temperature, the membranes were incubated overnight at 4 °C with agitation with the mouse monoclonal anti-TDP-43 3H8 (Novus, diluted 1:5000 in blocking buffer), with the rabbit monoclonal anti-phospho Ser410 TDP-43 (Sigma-Aldrich, diluted 1:1000 in blocking buffer) antibodies, or with HisProbe-HRP (ThermoFisher Scientific, diluted 1 :2000 in blocking buffer). The membranes were washed 3 times for 10 min each with TBS-T and then incubated with either horseradish peroxidase (HRP)-labeled goat anti266 mouse IgG (Sigma-Aldrich, 1:5000 dilution) for 3H8, or anti-rabbit IgG (Sigma-Aldrich,1:4000 dilution) for anti-phospho Ser410. After washing, the membranes were developed using Clarity Western-ECL (BioRad), and the signals were recorded with the ChemiDoc XRS-Plus imager (BioRad).

**1.11. Circular Dichroism (CD)**

CD spectra were recorded at 25 °C in a Jasco-810 CD spectrometer with 0.3 mg/ml protein solutions in 5 mM potassium phosphate/10 Mm NaCl [pH 6.8] using a 0.1 cm cuvette. For each measurement, six scans were averaged and, after correction for the buffer contribution, transformed into mean residue weight ellipticity ($\Theta_{mrw}$).

**1.12. Transmission Electron Microscopy (TEM) analysis of negatively stained samples.**

Samples containing 50 $\mu$M PLD and MetO PLD were incubated in incubation buffer at 37 °C in an orbital shaker operated at 300 rpm. For TEM specimen preparation, 5 $\mu$l of 3, 5, 10 and 20 days aged PLD and MetO PLD samples were placed onto carbon coated, formvar 300 mesh copper grids (Ted Pella) that were freshly glow discharged with a PELCO easyGlow instrument (Ted Pella). After 1 min incubation at room temperature, the solvent excess was blotted with filter paper (Whatman). The grids were next stained with 5 $\mu$l of 2% (w/v) uranyl acetate in water for 1 min. After blotting to remove the excess of stain, the grids were dried before imaging. Micrographs were acquired using a Gatan ORIUS SC600 (Model 831) digital camera on a FEI Tecnai G2 20 S-TWIN electron microscope that was operated at 200 kV.

### 1.13. AFM imaging

Samples containing 100 $\mu$M PLD and MetO PLD in incubation buffer were stored for 4 months at 25 °C with no agitation. 10 $\mu$l of 10 $\mu$M protein were added on top of a freshly cleaved mica and dried. Imaging was performed using an AFM microscope (Nanotec Electronica) in dynamic mode and HQ:NSC15/No Al cantilevers ($\mu$masch), with a spring constant of 40 N/m and a nominal resonance frequency of 330 kHz. Images were analyzed using WSxM software (Horcas I, Fernandez R, Gomez-Rodriguez JM, Colchero J, Gomez-Herrero J, Baro AM. WSXM: a software for scanning probe microscopy and a tool for nanotechnology. Rev Sci Instrum 78, 013705 (2007)).

### 2. Results

### 2.1. CNBr digestion confirms the presence of TDP-43 C-terminal sulfoxidized fragments in ALS spinal cord.

CNBr cleaves protein chains after Met residues with high efficiency. Efficient digestion of C-terminal fragments of TDP-43 with CNBr would degrade the chain into a variety of short peptides. If, otherwise, TDP-43 contains oxidized methionines, CNBr digestion would be compromised, and resistant chains will show reactivity in the western blot using the anti-phospho Ser410 TDP-43 antibody that recognizes a region encompassing the phosphorylated residues Ser 410. This antibody is commonly used to detect TDP-43 deposits in post-mortem material (Neumann M, et al. Ubiquitinated TDP-43 in frontotemporal lobar degeneration and amyotrophic lateral sclerosis. Science 314, 130-133 (2006)).

Presence of resistant bands indicate the failure of CNBr cleavage due to the TDP-43 methionine oxidation in the ALS spinal cord samples. Lack of reactivity in control samples indicate significant CNBr degradation of TDP-43 and/or absence of insoluble phosphorylated TDP-43 in healthy individuals **(Fig. 1).** This observation corroborates the presence of TDP-43 with chemically modified methionines (in particular, methionine sulfoxidation) in ALS patients.

This approach overcomes the limitations of mass spectrometry in the detection of TDP-43 C-terminal fragment, which in addition can induce artefactual modifications in the protein by sample handling. See for instance the lack of detection of TDP-43 in: Accurate Proteomewide Measurement of Methionine Oxidation in Aging Mouse Brains. Bettinger JQ, Simon M, Korotkov A, Welle KA, Hryhorenko JR, Seluanov A, Gorbunova V, Ghaemmaghami S. J Proteome Res. 2022 Jun 3;21(6):1495-1509. doi:10.1021/acs.jproteome.2c00127).

### 2.2. Methionine sulfoxidation impairs the association behavior of TDP-43 PLD

Besides well conserved structured domains responsible for oligomerization, nuclear export and RNA binding, TDP-43 (SEQ ID NO: 3) contains a disordered region at the C-terminus with prion-like properties **(Fig. 3A).** This PLD (SEQ ID NO: 2), which covers the region 274-414, contains most of the ALS-associated mutations, is rich in methionine residues and is sufficient to bolster the aggregation of full-length TDP-43 in disease. The PLD is able to promote condensates by LLPS *in vitro* and in cells, although with different aging properties relative to those condensates formed by full-length TDP-43 (Lu S, etal. Heat shock chaperone HSPB1 regulates cytoplasmic TDP-43 phase separation and liquid-to-gel transition. Nat Cell Biol 24, 1378-1393 (2022)). To validate LLPS by the PLD, the inventors monitored protein de-mixing by turbidimetry and optical microscopy **(Figs. 3B-F).** Turbidimetry showed that both TDP-43 and its PLD exhibited minimal phase separation at low concentrations (10-20 $\mu$M, **Figs. 3B-D).** At higher concentrations, PLD phase separated with a strong dependence on the type and concentration of salt. In presence of sodium ions, LLPS was significantly more effective, even at lower PLD concentrations **(Fig. 3B-F).** To determine the impact of disease-relevant post-translational modifications in PLD de-mixing, the PLD (amino acid sequence SEQ ID NO: 2) was incubated with $H_2O_2$ to achieve methionine sulfoxidation (termed MetO PLD, also named as MetO-PLD polypeptide of the invention). Remarkably, even in conditions that potently favor de-mixing (namely high protein and salt concentrations), MetO PLD displays an impaired phase separation **(Figs. 3D-G).**

NMR spectroscopy was employed to determine the structural output of methionine sulfoxidation on the PLD. While the NMR spectra of 300 $\mu$M PLD showed a highly disordered protein and contained signs of protein condensation **(Fig. 4A),** MetO causes significant changes in the NMR spectra **(Figs. 4B-C).** Crosspeaks belonging to methionine and nearby residues shift significantly **(Figs. 4C),** indicating a strong change in their chemical environment due to oxidation.

Assignment of the novel resonances showed that PLD Met residues are fully oxidized following treatment with $H_2O_2$, which is validated by the characteristic shifts in their C$\beta$ resonances. Remarkably, no oxidation of Trp/Phe residues, which are key stabilizing TDP-43 LLPS, was detected by NMR or mass spectrometry **(Table 1).**

**Table 1. List of modifications detected by mass spectrometry in MetO PLD.** Met residues are highlighted. The C-terminal Met residue (M414) was not detected. Considering the sequence properties of the PLD, long peptide digestions were necessary at neutral/alkaline pH, which can lead to artificial deamidation. In addition, no modifications in the N/Q residues present above were observed in the NMR spectra. Therefore, it was concluded that the deamidation modifications result from sample preparation.

| Residue (residues positions relative to the full human TDP-43 protein amino acid sequence -SEQ ID NO: 3-) | Post-translational Modification | Percentage |
|---|---|---|
| N285 | Deamidation | 99.7% |
| Q286 | Deamidation | 99.5% |
| N291 | Deamidation | 100% |
| N301 | Deamidation | 100% |
| N306 | Deamidation | 100% |
| **M307** | Oxidation | 100% |
| **M311** | Oxidation | 100% |
| N312 | Deamidation | 100% |
| N319 | Deamidation | 98.9% |
| **M322** | Oxidation | 75% |
| **M323** | Oxidation | 99.5% |
| Q331 | Deamidation | 98.7% |
| **M336** | Oxidation | 100% |
| **M337** | Oxidation | 100% |
| **M339** | Oxidation | 100% |
| Q346 | Deamidation | 97% |
| N352 | Deamidation | 99.7% |
| N353 | Deamidation | 95.4% |
| N358 | Deamidation | 100% |
| **M359** | Oxidation | 99.6% |
| N372 | Deamidation | 98.4% |
| N378 | Deamidation | 97,9% |
| N390 | Deamidation | 100% |
| N398 | Deamidation | 100% |
| **M405** | Oxidation | 100% |

NMR signal broadening, which is indicative of exchange processes in the NMR timescale in addition to intermolecular associations, shows that there is a strong broadening in the region including residues 305-345 of unmodified PLD upon phase separation **(Fig. 4D).** This observation indicates that, upon increasing protein concentrations, PLD self-association is mediated by this hydrophobic region. In addition, crosspeaks belonging to this particular region are absent in the CON NMR spectra acquired for PLD under LLPS conditions **(Fig. 5).** Since CON spectra are less affected by chemical exchange yet sensitive to fast transverse relaxation, this strongly suggests that the 305-345 region of PLD is involved in intermolecular interactions in the de-mixed phase. Comparison of the NMR signal broadening revealed that MetO PLD is less prone to self-assemble **(Fig. 4D),** confirming its decreased de-mixing. $^{15}$N spin relaxation param-

eters corroborated that the PLD contains rigid elements in its structure and that it is assembled in larger species, while MetO PLD remains highly dynamic and disperse even at high concentrations **(Table 2).**

**Table 2. Correlation time, order and exchange parameters for the region 321-343 of PLD and MetO PLD.** $S^2$ were derived from the chemical shifts, the overall correlation time ($\tau c$) from the ratio of the mean values of T1 and 72, and $k_{ex}$ and $p_B$ from $^{15}$N CPMG data. Increased $S^2$ and $\tau_C$ for PLD (unmodified PLD, with amino acid sequence SEQ ID NO: 2) indicate that the protein is less flexible and assembles into larger species, respectively, while MetO PLD (MetO-PLD polypeptide of the invention) is more flexible and remains disperse. MetO PLD showed no conformational exchange in the $\mu$s-ms time domain. Data is related specifically to the structured region of the PLD (comprising residues 321-343; residue's positions relative to TDP-43; SEQ ID NO: 3).

|  | $T_1$ (s) | $T_2$ (s) | (1H)-$^{15}$N NOE | $S^2$ | $\tau_C$ (ns) | $k_{ex}$ (s$^{-1}$) | $p_B$ |
|---|---|---|---|---|---|---|---|
| PLD | 0.30 +/- 0.07 | 0.13 +/- 0.05 | 0.56 +/- 0.15 | 0.51 +/- 0.17 | 3.3 | >2000 | 4 $\pm$ 0.5 % |
| MetO PLD | 0.26 +/- 0.02 | 0.17 +/- 0.02 | 0.38 +/- 0.09 | 0.40 +/- 0.13 | 1.5 | n.a. | n.a. |

In addition, $^{15}$N Carr-Purcell-Meiboom-Gill (CPMG)-based relaxation dispersion experiments (Wang C, Grey MJ, Palmer AG. CPMG sequences with enhanced sensitivity to chemical exchange. J Biomol NMR 21, 361-366 (2001)) confirmed that the PLD experienced a conformational exchange in the $\mu$s-ms chemical shift timescale, indicative of its assembly into dynamic ensembles **(Table 2).** Remarkably, lack of exchange for MetO PLD confirms that methionine sulfoxidation abrogates phase separation.

## 2.3. Methionine sulfoxidation promotes significant disorder

Because structured elements in TDP-43 PLD are critical for phase separation, inventors aimed to monitor any possible conformational transition upon protein de-mixing. The structural tendencies observed in PLD do not vary with increasing protein concentrations, regardless of the phase composition **(Figs. 4E).** In brief, there is a double $\alpha$-helix formed between residues 321-331 and 334-343, and there are small tendencies to adopt $\beta$-strand structures in the regions 368-381 and 391-399 of the PLD. The additional $\beta$-strand expected to form in the low-complexity aromatic-rich kinked segment (LARKS, region 312-317) (Bolognesi B, Faure AJ, Seuma M, Schmiedel JM, Tartaglia GG, Lehner B. The mutational landscape of a prion-like domain. Nat Commun 10, 4162 (2019)) is not apparent in our conditions. Therefore, the helical segments in PLD, which are present *in vivo,* appear equally populated in disperse samples, and no additional conformational transitions are observed upon phase de-mixing.

Upon oxidation, the $\alpha$-helical content is significantly decreased, as shown by NMR secondary chemical shifts **(Fig. 4E)** and circular dichroism (CD) analysis **(Fig. 6).** The structural data indicates that the $\alpha$-helices between residues 321-331 and 334-343 are largely dismantled in MetO PLD (MetO-PLD polypeptide of the invention), while the $\beta$-strand structures in the regions 368-381 and 391-399 remain intact **(Fig. 4E).** These structural effects correlate with the location of Met residues and the chemical shifts observed in MetO PLD and with the absence of rigid elements observed in MetO PLD $^{15}$N relaxation profiles **(Table 2).** Considering the key role of the $\alpha$-helices in PLD de-mixing, the conformational rearrangements help to explain the inability of MetO PLD to phase separate. Since fibril formation can be viewed as a liquid-to-solid transition in aging condensates, inventors next wondered whether the metamorphism displayed by MetO PLD affects aggregation.

## 2.4. Oxidized PLD assembles into ultrastructurally different fibrillar aggregates

PLD amyloid aggregates are sustained by a cross-$\beta$sheet skeleton in which the side chains of several Met residues are closely packed. Conversely, those Met residues lie on the solvent-exposed surfaces of the $\alpha$-helical segments in solution. Therefore, an increase in polarity and volume of methionine residues side-chains may cause a significant impact in PLD fibril formation. Contrary to the expectations pointing towards an impaired aggregation, full methionine sulfoxidation drastically delays but does not impede PLD fibrillar aggregation as monitored by Thioflavin T (ThT) fluorescence, irrespective of the salt composition **(Fig. 7A).** Kinetic analysis indicates that the aggregation of PLD and MetO PLD are governed by distinct mechanisms **(Fig. 7B-D, Table 3).**

**Table 3. Dependence of the time at which half of the monomer is converted to fibril, *t1/2,* on the amount of seeds present for PLD and MetO PLD.**

| % Seeds | $t_{1/2}$ (h) | |
| --- | --- | --- |
| | PLD | MetO PLD |
| 0 | 4.87 ± 0.37 | 54.88 ± 1.60 |
| 0.1 | 4.99 ± 0.31 | 38.26 ± 0.20 |
| 0.5 | 2.88 ± 0.06 | 18.70 ± 0.28 |
| 1 | 2.86 ± 0.15 | 18.02 ± 0.22 |

In particular, lack of lag phase and of a concentration effect on the $t_{1/2}$ in PLD aggregation suggests that the limiting step is a conformational transition towards amyloid-compatible structures, while methionine sulfoxidation severely impairs a primary nucleation event. In agreement with ThT measurements, transmission electron microscopy (TEM) showed that methionine sulfoxidation on the PLD monomer hinders the rate of fibril formation at physiological pH **(Fig. 8A)**. In addition, fibrils formed by MetO PLD appeared morphologically different from those formed by unmodified PLD at the tested time points **(Figs. 8A)**. Indeed, atomic force microscopy (AFM) shows that the aged amyloid fibrils formed by PLD are unbranched but densely entangled **(Figs. 8B)**. On the contrary, the aged fibrils formed by MetO PLD appear as extremely long, homogeneous and isolated assemblies **(Fig. 8B)**. These ultrastructural differences may entail the presence of alternative packing arrangements due to methionine sulfoxidation within the fibril core. All in all, methionine sulfoxidation causes a significant structural conversion in the PLD which suppresses phase separation and promotes self-association into seemingly alternative aggregates.

**2.5. Methionine sulfoxidation fully dismantles the second α-helix.**

Since MetO structural differences were clustered in the region including residues 312-346 of the PLD the segment 309-350 (termed PLD$_{309}$) (SEQ ID NO: 1) was used to determine the structure and relaxation properties of MetO PLD by NMR. Analysis of heteronuclear (1H)-15N nuclear Overhauser effect (NOE) and 15N longitudinal ($R_1$) and transverse ($R_2$) rate constants showed that MetO PLD$_{309}$ (MetO-PLD309 polypeptide of the invention) is highly dynamic in solution **(Fig. 9A)**. Only a minimal increase in the relaxation parameters in the region 327-334 suggests rigidity due to the formation of ordered structures. While this lack of apparent rigidity was corroborated by MetO PLD 15N relaxation parameters, it is in stark contrast with the backbone picosecond-nanosecond motions of unmodified PLD, which showed local rigidity in this particular region due to the formation of the double α-helix (Conicella AE, Zerze GH, Mittal J, Fawzi NL. ALS Mutations disrupt phase separation mediated by α-helical structure in the TDP-43 low-complexity C-terminal domain. Structure 24, 1537-1549 (2016)). NMR chemical shifts are also consistent with an overall high structural flexibility, particularly evident in the region where the second α-helix would be located (residues 334-343) **(Figs. 9B)**. In addition, J-coupling analysis showed lower values for the segment 322-331, indicating a tendency to acquire α-helical conformations in this region of MetO PLD$_{309}$ **(Fig. 9C)**. The rest of the protein showed 3J values clustering around 6 Hz, representative of a disordered conformational ensemble (Wang AC, Bax A. Determination of the backbone dihedral angles φ in human ubiquitin from reparametrized empirical Karplus equations. Journal of the American Chemical Society 118, 2483-2494 (1996)).

The increased flexibility of MetO PLD$_{309}$ precluded the observation of abundant 1H-1H NOE contacts that would define distance restraints for the structure calculation. Nonetheless, using chemical shifts and torsion angle restraints a set of 100 conformers were calculated by CYANA (Güntert P, Buchner L. Combined automated NOE assignment and structure calculation with CYANA. J Biomol NMR 62, 453-471 (2015).). The 20 conformers with the lowest target function were selected and assessed by PROCHECKNMR **(Fig. 9D)**. Direct comparison with the available solution structures of the 311-360 fragment from the PLD (PDB code 2n3x) (Jiang LL, et al. Two mutations G335D and Q343R within the amyloidogenic core region of TDP-43 influence its aggregation and inclusion formation. Sci Rep 6, 23928 (2016)) highlights the absence of the second α-helix due to the clustering of MetO336, MetO337 and MetO339. In addition, MetO322 and MetO323 cause the partial dismantling of the N-terminal part of the first α-helix. The high intrinsic dynamics of the short α-helix between residues Ala324-Ser332 reduces the structural convergence of the conformers obtained (RMSD to the mean coordinates of 0.41 Å for heavy backbone atoms). Indeed, considering the empirical Cα chemical shifts for 100% α-helical conformers (+3.1 ppm) (Spera S, Bax A. Empirical correlation between protein backbone conformation and Calpha and Cbeta 13C nuclear magnetic resonance chemical shifts. Journal of the American Chemical Society 113, 5490-5492 (1991)), the short α-helix acquired by MetO PLD$_{309}$ only populates about 27% of the conformational ensemble **(Fig. 9B)**.

**2.6. Methione sulfoxidation changes PLD interactome: Chaperones do not recognize MetO PLD**

The decline of protein misfolding management by molecular chaperones allows the manifestation of various protein aggregation diseases. HSP70 and HSP90 are the main cytoplasmic chaperones, and their refolding activities are tightly regulated by a large cohort of co-chaperones. HSP70, HSP90 and certain co-chaperones prevent the cytoplasmic aggregation of TDP-43 PLD. In particular, HSP70 is able to phase separate in combination with TDP-43 (Yu H, et al. HSP70 chaperones RNA-free TDP-43 into anisotropic intranuclear liquid spherical shells. Science 371, eabb4309 (2021); Gu J, et al. Hsp70 chaperones TDP-43 in dynamic, liquid-like phase and prevents it from amyloid aggregation. Cell Res 31, 1024-1027 (2021)), and its proper ATP-dependent activity may determine aberrant phase separation in stress. Because the levels of the inducible isoforms of HSP70 and HSP90 (HSP72 and HSP90$\alpha$ and HSP90$\beta$, respectively) substantially increase in stress, inventors analyzed their impact on PLD phase separation. All HSP70 and HSP90 isoforms promoted the de-mixing of PLD in a highly similar manner, in a way which resembles the LLPS of highly-concentrated PLD **(Fig. 10A)**.

Residue-by-residue analysis of the interactions established by the PLD and chaperones by NMR spectroscopy showed that chaperones recognized the region 321-343 in the PLD **(Fig. 10B)**. In particular, the four chaperones tested were able to bind in identical fashion to the double $\alpha$-helical motif in the PLD. Remarkably, the signal broadening observed in disperse PLD upon chaperone binding resembled very closely that observed in de-mixed PLD, suggesting that chaperones promote intermolecular associations in the PLD via the double $\alpha$-helical motif. On the contrary, recognition of the PLD by HSP70 and HSP90 chaperones was suppressed by methionine sulfoxidation **(Fig. 10C)**. Since chaperones bind to the structured regions of the PLD **(Fig. 10B)**, impairment of both hydrophobicity and $\alpha$-helical motifs in MetO PLD **(Fig. 9D)** dictates the evasion from chaperone recognition and commands its accumulative trend.

**2.7. Methione sulfoxidation changes PLD interactome: CK1$\delta$ phosphorylates mainly aggregated MetO PLD**

The presence of hyperphosphorylated TDP-43 chains is the pathognomonic feature of the inclusions isolated from ALS and FTLD patients (Neumann M, et al. Ubiquitinated TDP-43 in frontotemporal lobar degeneration and amyotrophic lateral sclerosis. Science 314, 130-133 (2006)). However, because the core of the fibrillar amyloid aggregates obtained from the brain of patients do not show phosphorylation modifications (Arseni D, et al. Structure of pathological TDP-43 filaments from ALS with FTLD. Nature 601, 139-143 (2022)), it is unclear whether phosphorylation occurs in the pre-formed aggregates or in a later stage, as an attempt to eliminate the aggregates. Alternatively, phosphorylation may affect TDP-43 balanced phase separation, providing a basis for the prevalence of phosphorylation traits in disease and its potential correlation with disease subtypes. Therefore, determining the structural impact of phosphorylation on the PLD and its crosstalk with methionine oxidation can provide relevant insights into the role of modifications in protein diseases.

Casein kinase-1 (CK1$\delta$) has been postulated as one of the kinases involved in the hyperphosphorylation of TDP-43 *in vivo* (Kametani F, et al. Identification of casein kinase-1 phosphorylation sites on TDP-43. Biochem Biophys Res Commun 382, 405-409 (2009); Choksi DK, et al. TDP-43 Phosphorylation by casein kinase I$\varepsilon$ promotes oligomerization and enhances toxicity in vivo. Hum Mol Genet 23, 1025-1035 (2014)). Phosphorylation of the PLD by CK1$\delta$ was confirmed by immunoblotting with anti-phospho Ser410 TDP-43 antibody (also named in the present document as pS410 antibody), which recognizes phosphorylated TDP43 Ser410, located in the PLD of the TDP-43 amino acid sequence (Ser410 of SEQ ID NO: 3) **(Fig. 11A)**. Interestingly, significant aggregation was evident upon phosphorylation, detected by the presence of high molecular weight bands **(Fig. 11B)**. In addition, pS410 antibody revealed that phosphorylation was drastically diminished in soluble MetO PLD but becomes significant after its amyloid assembly **(Fig. 11A and B)**.

**2.8. Methione sulfoxidation changes PLD interactome: MetO PLD interacts with and amplifies aggregates present in the CSF of ALS patients.**

As shown in **Fig. 7,** both PLD and MetO PLD aggregate into amyloid fibrils following different mechanism. In particular, kinetics showed that nucleation is the limiting step in the aggregation of MetO PLD and that the time at which half of the monomer is converted into fibril is highly sensitive to the presence of preformed aggregates or seeds (propagons). Presence of CSF as seed from control and ALS patients differently modifies the kinetics of MetO PLD aggregation in media containing 0.4M HClGdn **(Fig. 2A)** or 0.5M urea **(Fig. 2B).** At 30 h, increases in the ThT fluorescence are observed for the CSF of ALS patients whereas the CSF from controls remain at the base line level.

Comparison with the control seeds experiments indicate that MetO PLD (MetO-PLD polypeptide of the invention; SEQ ID NO: 2 amino acid sequence with oxidized methione residues) is an ideal substrate for probing propagon reactivity in terms of signal sensitivity and significance, and also in terms of stability and homogeneity of the substrate preservation conditions.

These results validate the use of MetO PLD as optimal reagent to test the presence of aggregated PLD in samples with high selectivity.

**Claims**

1. Polypeptide comprising the amino acid sequence SEQ ID NO: 1, wherein, at least, 80% of the methionine residues in said amino acid sequence SEQ ID NO: 1 are oxidized.

2. Polypeptide according to claim 1, comprising the amino acid sequence SEQ ID NO: 2, wherein, at least, 80% of the methionine residues in said amino acid sequence SEQ ID NO: 2 are oxidized.

3. Polypeptide comprising the amino acid sequence SEQ ID NO: 3, which in turn comprises SEQ ID NO: 1, wherein, at least, 80% of the methionine residues of SEQ ID NO: 1 are oxidized.

4. Polypeptide comprising the amino acid sequence SEQ ID NO: 3, which in turn comprises SEQ ID NO: 2, wherein, at least, 80% of the methionine residues of SEQ ID NO: 2 are oxidized.

5. Use of the polypeptide according to any one of claims 1 to 4, as a therapeutic target for designing and/or screening molecules, compounds, compositions, drugs or the like, useful for the treatment and/or prevention of a TDP-43 proteinopathy.

6. Use according to claim 5, wherein the TDP-43 proteinopathy is amyotrophic lateral sclerosis (ALS).

7. Use according to claim 5, wherein the TDP-43 proteinopathy is frontotemporal lobar degeneration (FTLD).

8. Use of the polypeptide according to any one of claims 1 to 4, as a target for designing and/or screening molecules, compounds, compositions, drugs or the like, useful for the detection of methionine oxidized TDP-43 proteins and/or fragments thereof.

9. Use of the polypeptide according to any one of claims 1 to 4 as a detection reagent for imaging TDP-43 aggregates.

10. Method for the screening of prophylactic and/or therapeutic compounds for a TDP-43 proteinopathy, which comprises the following steps:

> a) preparing a first reaction mixture, wherein said reaction mixture comprises a previously isolated biological sample from a biological system treated with a test compound, and an amyloid-specific probe, wherein said probe is able to produce a detectable signal;
> b) preparing a second reaction mixture, wherein said reaction mixture comprises a previously isolated biological sample from a biological system not treated with a test compound, and an amyloid-specific probe, wherein said probe is able to produce a detectable signal;
> c) adding the polypeptide according to any one of claims 1 to 4, to the reaction mixture prepared in step a), obtaining a reaction mixture (i), and to the reaction mixture prepared in step b), obtaining a control reaction mixture (ii);
> d) an amplification step, which comprises incubating with agitation the reaction mixture (i), and the control reaction mixture (ii), preferably, wherein said agitation is carried out in presence of beads;
> e) measuring, simultaneously to when the amplification step d) is performed, the detectable signal in the reaction mixture (i) and the control reaction mixture (ii); and
> f) selecting, as a prophylactic and/or therapeutic candidate compound for a TDP-43 proteinopathy, a test compound that changes the measured signal in the reaction mixture (i) compared to the measured signal in the control reaction mixture (ii).

11. Method according to claim 10, wherein the TDP-43 proteinopathy is ALS.

12. Method according to claim 10, wherein the TDP-43 proteinopathy is FTLD.

13. Method for the *in vitro* diagnosis of a TDP-43 proteinopathy in an isolated biological sample from a subject, which comprises the following steps:

a) preparing a reaction mixture, wherein said reaction mixture comprises the isolated biological sample, preferably wherein the isolated biological sample is selected from the list consisting of a cerebrospinal fluid sample, a serum sample, a spinal-cord sample, a muscle sample and a cortex sample, and an amyloid-specific probe, wherein said probe is able to produce a detectable signal;

b) adding the polypeptide according to any one of claims 1 to 4, to the reaction mixture prepared in step a);

c) an amplification step, which comprises incubating with agitation the reaction mixture obtained in step b), preferably, wherein said agitation is carried out in presence of beads;

d) measuring, simultaneously to when the amplification step c) is performed, the detectable signal;

e) comparing the detectable signal with reference values, wherein an intensity increase in the measured detectable signal and/or a decrease of the time at which half of the maximum intensity of the signal is reached, compared to the reference value, indicates that the subject has pre-formed TDP-43 propagons and, therefore, a TDP-43 proteinopathy.

14. Method according to claim 13, wherein the TDP-43 proteinopathy is ALS.

15. Method according to claim 13, wherein the TDP-43 proteinopathy is FTLD.

Fig. 1

Fig. 2

Fig.3

E

F

PLD 150μM @ 3h

G

MetO PLD 150μM / NaCl @ 48h

**Fig. 3 (cont.)**

**Fig.4**

**Fig. 4 (cont.)**

**Fig. 5**

**Fig. 6**

**Fig. 7**

**Fig. 8**

Fig. 9

**Fig. 10**

**Fig. 11**

Europäisches
Patentamt

European
Patent Office

Office européen
des brevets

# EUROPEAN SEARCH REPORT

Application Number

EP 22 38 3245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2022/010761 A1 (UNIV TEXAS [US]) 13 January 2022 (2022-01-13) * see claims 1-27 The query sequence SEQ ID NO:1 has 100.00 % identity (100.00 % similarity) over 42 positions in a common overlap (range (q:s): 1-42:47-88) with subject GSP:BKN70566 (length: 152) from WO2022010761-A1 published on 2022-01-13. * | 1-15 | INV. A61P25/00 A61P25/02 A61P25/28 G01N33/68 |
| Y,D | WO 2009/044119 A1 (UNIV MANCHESTER [GB]; PICKERING-BROWN STUART [GB] ET AL.) 9 April 2009 (2009-04-09) * see claims 1-15 * | 1-15 | |
| Y | WO 2018/204764 A1 (CAMP4 THERAPEUTICS CORP [US]) 8 November 2018 (2018-11-08) * The query sequence SEQ ID NO:1 has 100.00 % identity (100.00 % similarity) over 42 positions in a common overlap (range (q:s): 1-42:10-51) with subject GSP:BFV27009 (length: 115) from WO2018204764-A1 published on 2018-11-08. * | 1-15 | |
| Y,D | ARSENI DIANA ET AL: "Structure of pathological TDP-43 filaments from ALS with FTLD", NATURE, NATURE PUBLISHING GROUP UK, LONDON, vol. 601, no. 7891, 8 December 2021 (2021-12-08), pages 139-143, XP037656909, ISSN: 0028-0836, DOI: 10.1038/S41586-021-04199-3 [retrieved on 2021-12-08] * the whole document * | 1-15 | |

TECHNICAL FIELDS
SEARCHED (IPC)

A61P
G01N

-/--

The present search report has been drawn up for all claims

| Place of search | Date of completion of the search | Examiner |
|---|---|---|
| Munich | 12 May 2023 | Vix, Olivier |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

# EUROPEAN SEARCH REPORT

**Application Number**

EP 22 38 3245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | KAMETANI FUYUKI ET AL: "Mass spectrometric analysis of accumulated TDP-43 in amyotrophic lateral sclerosis brains", SCIENTIFIC REPORTS, vol. 6, no. 1, 1 September 2016 (2016-09-01), XP055806601, DOI: 10.1038/srep23281 Retrieved from the Internet: URL:https://www.nature.com/articles/srep23281.pdf> * the whole document * | 1-15 | |
| Y | GUENTHER ELIZABETH L ET AL: "Atomic structures of TDP-43 LCD segments and insights into reversible or pathogenic aggregation", NATURE STRUCTURAL & MOLECULAR BIOLOGY, NATURE PUBLISHING GROUP US, NEW YORK, vol. 25, no. 6, 21 May 2018 (2018-05-21), pages 463-471, XP036888316, ISSN: 1545-9993, DOI: 10.1038/S41594-018-0064-2 [retrieved on 2018-05-21] * the whole document * | 1-15 | TECHNICAL FIELDS SEARCHED (IPC) |
| Y | GIORGIO COLOMBO ET AL: "Methionine Sulfoxides on Prion Protein Helix-3 Switch on the α-Fold Destabilization Required for Conversion", PLOS ONE, vol. 4, no. 1, 1 January 2009 (2009-01-01) , pages e4296-e4296, XP055077744, ISSN: 1932-6203, DOI: 10.1371/journal.pone.0004296 * the whole document * | 1-15 | |

-/--

The present search report has been drawn up for all claims

| Place of search | Date of completion of the search | Examiner |
|---|---|---|
| Munich | 12 May 2023 | Vix, Olivier |

Europäisches
Patentamt

European
Patent Office

Office européen
des brevets

# EUROPEAN SEARCH REPORT

Application Number

EP 22 38 3245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | CONICELLA ALEXANDER E ET AL: "ALS Mutations Disrupt Phase Separation Mediated by [alpha]-Helical Structure in the TDP-43 Low-Complexity C-Terminal Domain", STRUCTURE, vol. 24, no. 9, 6 September 2016 (2016-09-06), pages 1537-1549, XP029725077, ISSN: 0969-2126, DOI: 10.1016/J.STR.2016.07.007 * a perfectly conserved region spanning residues 319-341, rich in aliphatic residues (7 alanine, 5 methionine, 2 leucine) nearly absent in the remaining 120 residues of the domain. * ----- | 1-15 | |
| Y | BAO CHANNA ET AL: "Mechanisms of Regulation and Diverse Activities of Tau-Tubulin Kinase (TTBK) Isoforms", CELLULAR AND MOLECULAR NEUROBIOLOGY, vol. 41, no. 4, 18 May 2020 (2020-05-18), pages 669-685, XP037411613, ISSN: 0272-4340, DOI: 10.1007/S10571-020-00875-6 * In phosphoprotemics study for action of Tau-Tubulin kinase (TTBK) , MS/MS spectra searches allowed variable modifications of oxidized methionine,phosphorylation (STY), N-terminal acetylation and deamidation (NQ).; page 672 - page 673 * ----- | 1-15 | TECHNICAL FIELDS SEARCHED (IPC) |
| A | WO 2020/234473 A1 (AC IMMUNE SA [CH]) 26 November 2020 (2020-11-26) * see claims 1-57 * ----- | 1-15 | |

-/--

The present search report has been drawn up for all claims

| Place of search | Date of completion of the search | Examiner |
|---|---|---|
| Munich | 12 May 2023 | Vix, Olivier |

EPO FORM 1503 03.82 (P04C01)

Europäisches
Patentamt

European
Patent Office

Office européen
des brevets

# EUROPEAN SEARCH REPORT

Application Number

EP 22 38 3245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EMILIEN FOLZER ET AL: "Selective Oxidation of Methionine and Tryptophan Residues in a Therapeutic IgG1 Molecule", JOURNAL OF PHARMACEUTICAL SCIENCES, vol. 104, no. 9, 1 September 2015 (2015-09-01), pages 2824-2831, XP055641164, US ISSN: 0022-3549, DOI: 10.1002/jps.24509 * the whole document * | 1-15 | |

TECHNICAL FIELDS SEARCHED (IPC)

The present search report has been drawn up for all claims

| Place of search | Date of completion of the search | Examiner |
|---|---|---|
| Munich | 12 May 2023 | Vix, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.............................................................................
& : member of the same patent family, corresponding document

EPO FORM 1503 03.82 (P04C01)

# EP 4 389 215 A1

## ANNEX TO THE EUROPEAN SEARCH REPORT
## ON EUROPEAN PATENT APPLICATION NO.

EP 22 38 3245

12-05-2023

| Patent document cited in search report | Publication date | Patent family member(s) | Publication date |
|---|---|---|---|
| WO 2022010761 A1 | 13-01-2022 | EP 4175716 A1 | 10-05-2023 |
| | | WO 2022010761 A1 | 13-01-2022 |
| WO 2009044119 A1 | 09-04-2009 | NONE | |
| WO 2018204764 A1 | 08-11-2018 | US 2021254056 A1 | 19-08-2021 |
| | | WO 2018204764 A1 | 08-11-2018 |
| WO 2020234473 A1 | 26-11-2020 | AU 2020278907 A1 | 20-01-2022 |
| | | BR 112021023487 A2 | 08-02-2022 |
| | | CA 3137882 A1 | 26-11-2020 |
| | | CL 2021003027 A1 | 24-06-2022 |
| | | CN 114008073 A | 01-02-2022 |
| | | EP 3972692 A1 | 30-03-2022 |
| | | IL 288314 A | 01-01-2022 |
| | | JP 2022533432 A | 22-07-2022 |
| | | KR 20220012270 A | 03-02-2022 |
| | | SG 11202112453T A | 30-12-2021 |
| | | TW 202110879 A | 16-03-2021 |
| | | US 2022315648 A1 | 06-10-2022 |
| | | WO 2020234473 A1 | 26-11-2020 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013108926 A1
- WO 2009044119 A1

**Non-patent literature cited in the description**

- **JOSEPHS KA et al.** TDP-43 is a key player in the clinical features associated with Alzheimer's disease. *Acta Neuropathol,* 2014, vol. 127, 811-824
- **PORTA S et al.** Distinct brain-derived TDP-43 strains from FTLD-TDP subtypes induce diverse morphological TDP-43 aggregates and spreading patterns in vitro and in vivo. *Neuropathol Appl Neurobiol,* 2021, vol. 47, 1033-1049
- **POLYMENIDOU M ; CLEVELAND DW.** The seeds of neurodegeneration: prion-like spreading in ALS. *Cell,* 2011, vol. 147, 498-508
- **ARSENI D et al.** Structure of pathological TDP-43 filaments from ALS with FTLD. *Nature,* 2022, vol. 601, 139-143
- **BABINCHAK WM et al.** The role of liquid-liquid phase separation in aggregation of the TDP-43 low-complexity domain. *J Biol Chem,* 2019, vol. 294, 6306-6317
- **ALBERTI S ; HYMAN AA.** Are aberrant phase transitions a driver of cellular aging?. *Bioessays,* 2016, vol. 38, 959-968
- **FANG YS et al.** Full-length TDP-43 forms toxic amyloid oligomers that are present in frontotemporal lobar dementia-TDP patients. *Nat Commun,* 2014, vol. 5, 4824
- **MATEJU D et al.** An aberrant phase transition of stress granules triggered by misfolded protein and prevented by chaperone function. *EMBO J,* 2017, vol. 36, 1669-1687
- **ARNOLD ES et al.** ALS-linked TDP-43 mutations produce aberrant RNA splicing and adult-onset motor neuron disease without aggregation or loss of nuclear TDP-43. *Proc Natl Acad Sci USA,* 2013, vol. 110, E736-745
- **KAMETANI F et al.** Mass spectrometric analysis of accumulated TDP-43 in amyotrophic lateral sclerosis brains. *Sci Rep,* 2016, vol. 6, 23281
- **PAGANONI, S. et al.** *Amyotroph. Lat. Scler. Fronto. Degener.,* 2014, vol. 15 (0), 453-6
- **ALTSCHUL S.F et al.** Basic local alignment search tool. *J Mol Biol.,* 05 October 1990, vol. 215 (3), 403-10

- **ALIYAN A. ; COOK N.P. ; ANGEL A. ; MARTÍ, A:A.** Interrogating Amyloid Aggregates using Fluorescent Probes. *Chemical Reviews,* 2019, vol. 119 (23), 11819-11856
- **ALIYAN A. ; COOK N.P ; ANGEL A. ; MARTÍ, A.A.** Interrogating Amyloid Aggregates using Fluorescent Probes. *Chemical Reviews,* 2019, vol. 119 (23), 11819-11856
- **PEREZ-BERLANGA et al.** *Bio Protoc,* 2019, vol. 9 (22), e3424
- **MARTINEZ et al.** *PLoS One,* 2011, vol. 6, e27999
- **MARTINEZ J et al.** Selenomethionine incorporation into amyloid sequences regulates fibrillogenesis and toxicity. *PloS One,* 2011, vol. 6, e27999
- **MARKLEY JL et al.** Recommendations for the presentation of NMR structures of proteins and nucleic acids. IUPAC-IUBMB-IUPAB Inter-Union Task Group on the Standardization of Data Bases of Protein and Nucleic Acid Structures Determined by NMR Spectroscopy. *J Biomol NMR,* 1998, vol. 12, 1-23
- **KJAERGAARD M ; BRANDER S ; POULSEN FM.** Random coil chemical shift for intrinsically disordered proteins: effects of temperature and pH. *J Biomol NMR,* 2011, vol. 49, 139-149
- **VUISTER GW ; DELAGLIO F ; BAX A.** The use of 1JC alpha H alpha coupling constants as a probe for protein backbone conformation. *J Biomol NMR,* 1993, vol. 3, 67-80
- **WANG C ; GREY MJ ; PALMER AG.** CPMG sequences with enhanced sensitivity to chemical exchange. *J Biomol NMR,* 2001, vol. 21, 361-366
- **BIERI M ; GOOLEY PR.** Automated NMR relaxation dispersion data analysis using NESSY. *BMC Bioinformatics,* 2011, vol. 12, 421
- **SHEN Y ; BAX A.** Protein backbone and sidechain torsion angles predicted from NMR chemical shifts using artificial neural networks. *J Biomol NMR,* 2013, vol. 56, 227-241

- **KAY LE ; TORCHIA DA ; BAX A.** Backbone dynamics of proteins as studied by 15N inverse detected heteronuclear NMR spectroscopy: application to staphylococcal nuclease. *Biochemistry,* 1989, vol. 28, 8972-8979
- **PAWLEY NH ; WANG C ; KOIDE S ; NICHOLSON LK.** An improved method for distinguishing between anisotropic tumbling and chemical exchange in analysis of 15N relaxation parameters. *J Biomol NMR,* 2001, vol. 20, 149-165
- **LEE W ; TONELLI M ; MARKLEY JL.** NMR-FAM-SPARKY: enhanced software for biomolecular NMR spectroscopy. *Bioinformatics,* 2015, vol. 31, 1325-1327
- **KORADI R ; BILLETER M ; WUTHRICH K.** MOL-MOL: a program for display and analysis of macromolecular structures. *J Mol Graph,* 1996, vol. 14 (51-55), 29-32
- **HORCAS I ; FERNANDEZ R ; GOMEZ-RODRIGU-EZ JM ; COLCHERO J ; GOMEZ-HERRERO J ; BARO AM.** WSXM: a software for scanning probe microscopy and a tool for nanotechnology. *Rev Sci Instrum,* 2007, vol. 78, 013705
- **NEUMANN M et al.** Ubiquitinated TDP-43 in frontotemporal lobar degeneration and amyotrophic lateral sclerosis. *Science,* 2006, vol. 314, 130-133
- **BETTINGER JQ ; SIMON M ; KOROTKOV A ; WELLE KA ; HRYHORENKO JR ; SELUANOV A ; GORBUNOVA V ; GHAEMMAGHAMI S.** Accurate Proteomewide Measurement of Methionine Oxidation in Aging Mouse Brains. *J Proteome Res.,* 03 June 2022, vol. 21 (6), 1495-1509
- **LU S et al.** Heat shock chaperone HSPB1 regulates cytoplasmic TDP-43 phase separation and liquid-to-gel transition. *Nat Cell Biol,* 2022, vol. 24, 1378-1393
- **BOLOGNESI B ; FAURE AJ ; SEUMA M ; SCHMIEDEL JM ; TARTAGLIA GG ; LEHNER B.** The mutational landscape of a prion-like domain. *Nat Commun,* 2019, vol. 10, 4162
- **CONICELLA AE ; ZERZE GH ; MITTAL J ; FAWZI NL.** ALS Mutations disrupt phase separation mediated by $\alpha$-helical structure in the TDP-43 low-complexity C-terminal domain. *Structure,* 2016, vol. 24, 1537-1549
- **WANG AC ; BAX A.** Determination of the backbone dihedral angles $\varphi$ in human ubiquitin from reparametrized empirical Karplus equations. *Journal of the American Chemical Society,* 1996, vol. 118, 2483-2494
- **GÜNTERT P ; BUCHNER L.** Combined automated NOE assignment and structure calculation with CYANA. *J Biomol NMR,* 2015, vol. 62, 453-471
- **JIANG LL et al.** Two mutations G335D and Q343R within the amyloidogenic core region of TDP-43 influence its aggregation and inclusion formation. *Sci Rep,* 2016, vol. 6, 23928
- **SPERA S ; BAX A.** Empirical correlation between protein backbone conformation and Calpha and Cbeta 13C nuclear magnetic resonance chemical shifts. *Journal of the American Chemical Society,* 1991, vol. 113, 5490-5492
- **YU H et al.** HSP70 chaperones RNA-free TDP-43 into anisotropic intranuclear liquid spherical shells. *Science,* 2021, vol. 371, eabb4309
- **GU J et al.** Hsp70 chaperones TDP-43 in dynamic, liquid-like phase and prevents it from amyloid aggregation. *Cell Res,* 2021, vol. 31, 1024-1027
- **KAMETANI F et al.** Identification of casein kinase-1 phosphorylation sites on TDP-43. *Biochem Biophys Res Commun,* 2009, vol. 382, 405-409
- **CHOKSI DK et al.** TDP-43 Phosphorylation by casein kinase I$\epsilon$ promotes oligomerization and enhances toxicity in vivo. *Hum Mol Genet,* 2014, vol. 23, 1025-1035